Europäisches Patentamt

European Patent Office

Office européen des brevets

(11) Publication number: **0 247 843 B1**

# EUROPEAN PATENT SPECIFICATION

(45) Date of publication of patent specification: **08.09.93**

(51) Int. Cl.⁵: **C07K 13/00**, C07H 21/04, C12N 1/21, C12N 5/10, C12N 15/12, C12P 21/02, C12N 9/64, A61K 37/02, //(C12N1/20,C12R1:19)

(21) Application number: **87304676.7**

(22) Date of filing: **27.05.87**

(54) **Human protein S, A plasma protein regulator of hemostasis.**

(30) Priority: **27.05.86 US 866662**

(43) Date of publication of application:
**02.12.87 Bulletin 87/49**

(45) Publication of the grant of the patent:
**08.09.93 Bulletin 93/36**

(84) Designated Contracting States:
**BE CH DE FR GB IT LI NL SE**

(56) References cited:
**EP-A- 0 200 421**
**EP-A- 0 255 771**

**BIOCHEMISTRY, vol. 18, no. 5, 1979, pages 899-904, American Chemical Society, Washington, DC, US; R.G. DISCIPIO et al.: "Characterization of protein S, a gamma-carboxyglutamic acid containing protein from bovine and human plasma"**

(73) Proprietor: **ELI LILLY AND COMPANY**
**Lilly Corporate Center**
**Indianapolis Indiana 46285(US)**

(72) Inventor: **Hoskins, Jo Ann**
**8229 Tern Court**
**Indianapolis Indiana 46256(US)**
Inventor: **Long, George Louis**
**40 East Terrace Street**
**South Burlington Vermont 05403(US)**

(74) Representative: **Hudson, Christopher Mark et al**
**Erl Wood Manor**
**Windlesham Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.6/3.3.1)

GENE, vol. 37, no. 2-3, 1985, pages 215-220, Elsevier Science Publishers, Amsterdam, NL; B. LAPEYRE et al.: "A powerful method for the preparation of cDNA libraries: isolation of cDNA encoding a 100-kDal nucleolar protein"

PROCEEDINGS OF THE NATIONAL ACADEMY OF SCIENCE USA, vol. 84, January 1987, pages 349-353, Washington, DC, US; J. HOSKINS et al.: "Cloning and characterization of human liver cDNA encoding a protein S precursor"

PROCEEDINGS OF THE NATIONAL ACADEMY OF SCIENCE USA, vol. 83, September 1986, pages 6716-6720, Washington, DC, US; A.LUNDWALL et al.: "Isolation and sequence of the cDNA for human protein S, a regulator of blood coagulation"

PROCEEDINGS OF THE NATIONAL ACADEMY OF SCIENCE USA, vol. 83, June 1986, pages 4199-4203, Washington, DC, US; B. DAHL-BÄCK et al.: "Primary structure of bovine vitamin K-dependent protein S"

JOURNAL OF CLINICAL INVESTIGATION, vol. 74, December 1984, pages 2082-2088, The American Society for Clinical Investigation, Inc., New York, US; P.C. COMP et al.: "Familial protein S deficiency is associated with recurrent thrombosis"

**Description**

Thrombin formation, the conversion of pro-thrombin to thrombin, is regulated by a series of reactions, known as the coagulation cascade, that are catalyzed by serine proteases. These proteases depend upon a number of cofactor proteins, such as thrombomodulin, factors Va and VIIIa, and protein S, for maximal activity. Protein S is an important component in the recently discovered protein C-protein S-thrombomodulin system of major importance in the down-regulation of coagulation and intravascular thrombosis. This particular vitamin K-dependent plasma protein is required as a cofactor for optimal activated protein C (APC)-mediated inactivation of Factor Va on phospholipid vesicles and the platelet surface.

Protein S is a plasma protein which depends on vitamin K for its biosynthesis. The human protein possesses an apparent $M_r$ of 69-85 kilodaltons (Kd) and like other vitamin K-dependent proteins for biological function, it requires extensive post-translational modifications, including the conversion of eleven glutamic acid residues clustered towards the amino-terminus into gamma($\gamma$)-carboxyglutamic acid (Gla), the conversion of three aspartic acid residues (tentatively assigned herein to amino acid residues 95, 135, and 182) into beta-hydroxyaspartic acid, glycosylation (8% weight) and disulfide bond formation. The proposed function of the gamma-carboxyglutamic acid residues is to bind calcium which in turn facilitates the attachment of the molecule to phospholipid bilayer membranes; the function of the beta-hydroxyaspartic acid is unknown. Upon sodium dodecyl sulfate polyacrylamide gel electrophoresis (SDS PAGE) under reducing conditions, a heavy chain of 75 Kd and a light chain of 16 Kd emerges. Upon incubation with thrombin, an 8 Kd fragment is cleaved from the molecule resulting in loss of activity.

To define the role of protein S, a brief outline of this anticoagulant system is provided. Protein C, like protein S, depends on vitamin K for its biosynthesis and undergoes the Glu→Gla and the Asp→beta-hydroxyaspartic acid post-translational modifications. Protein C, a normal plasma protein, circulates as a serine protease zymogen. The physiological activator of protein C is thrombin complexed with an endothelial cell membrane glycoprotein, thrombomodulin. Free thrombin is a major coagulant protein; it converts fibrinogen to fibrin, activates platelets and the major cofactors in the clotting cascade, factors Va and VIIIa, thereby providing an important positive feedback loop potently amplifying the coagulation response. In contrast, free thrombin is a slow and ineffective activator of protein C. When complexed with thrombomodulin, thrombin strikingly changes its spectrum of enzymatic activities. Thrombin in complex with thrombomodulin no longer converts fibrinogen to fibrin, activates platelets, or activates clotting factors Va and VIIIa. Instead, thrombo-modulin-thrombin becomes a highly efficient activator of protein C. Activated protein C has two points of attack. It proteolytically degrades and inactivates the activated forms of cofactors Va and VIIIa, whereas the precursor forms of cofactors Va and VIIIa are very poor substrates for activated protein C. Protein S is an important cofactor for activated protein C. In the absence of protein S, activated protein C only minimally degrades and inactivates cofactors Va and VIIIa.

These complex reactions first established in vitro have important clinical corollaries. Homozygous protein C deficiency results in development of purpura fulminans, a lethal form of disseminated intravascular coagulation occurring in infancy or early childhood. The number of case reports of heterozygous protein C and especially protein S deficiency associated with deep vein thrombosis and recurrent pulmonary embolism are rapidly mounting and a preliminary estimate suggests that 10% of all cases of recurrent deep vein thrombosis-pulmonary embolism represent heterozygous protein S deficiency.

Moreover, recent evidence strongly suggests that acquired protein S deficiency may be a common disorder. Protein S exists in the circulation in two forms: as free protein S and in a one-to-one complex with C4b-binding protein. C4b-binding protein possesses a $M_r$ of 570 Kd and serves an important function in down-regulating the complement system in the fluid phase and on cell surfaces. Protein S complexed with the C4b-binding protein has no known biological activity. In pregnancy and immediately post-partum, a shift from free to bound forms of protein S occurs with a sharp decrease in functional protein S activity. In the nephrotic syndrome, the proportion of protein S complexed with C4b-binding protein is increased and the levels of free protein S are significantly reduced. In addition, for reasons not completely clear at this time, the specific activity of free protein S in the nephrotic syndrome is substantially reduced. In systemic lupus erythematosus (SLE) in which complement activation is present, a shift from free to bound protein S is again observed. Each of these disease states are associated with a high incidence of thromboembolism. The shift from free to bound protein S then may represent a control point at which inflammation or other physiological changes can directly influence the hemostatic balance. A less drastic but still significant decrease in free, biologically active protein S has also been observed in disseminated intravascular coagulation and liver disease.

Although the binding of protein S to the C4b-binding protein abolishes protein S activity, it does not appear that the complex loses C4b-binding protein activity. However, it is tempting to speculate that protein S with its gamma-carboxyglutamic acid residues designed to anchor this protein to phospholipid bilayer cell membranes may serve to transport the C4b-binding protein to membrane sites of complement down regulation.

Protein S has been isolated from both human and bovine plasma. The bovine protein S consists of a single polypeptide chain with a reported molecular weight of 64 Kd. In contrast, the apparent molecular weight of human protein S is reported to be 69-85 Kd. While methods are available for the isolation of protein S from plasma, until the present invention, very little was known about the primary or secondary structure of protein S. Notwithstanding the lack of information concerning protein S, the biological and potential therapeutic importance of protein S can be deduced from clinical observations. Presently available information indicates that hereditary protein S deficiency may be a common disorder. Protein S-deficient patients are predisposed to venous thrombotic disease at a relatively young age. Heterozygotes are at risk for superficial thrombophlebitis, deep vein thrombosis and pulmonary embolism. Given the severe consequences of protein S deficiency, it would be extremely advantageous to clone and express the gene for human protein S for use as a valuable adjunct in antithrombotic therapy.

Definitions

ApR - the ampicillin-resistant phenotype or gene conferring same.

ep - a DNA segment comprising the SV40 early promoter of the T-antigen (A) gene, the T-antigen binding sites, and the SV40 origin of replication.

Expression Vector - any recombinant DNA cloning vector into which a promoter has been incorporated and positioned to drive transcription of a desired gene.

Human Protein S - A precursor polypeptide including intermediate forms such as pro-protein S, descarboxy protein S, non-glycosylated protein S, and the mature protein, wherein all forms of the protein demonstrate protein S activity.

HmR - the hygromycin-resistant phenotype or gene conferring same.

IVS - a DNA encoding an intron, also called an intervening sequence.

NeoR - the neomycin-resistant phenotype or gene conferring same.

Ori - a plasmid origin of replication.

pA - a DNA sequence encoding a polyadenylation signal.

Promoter - a DNA sequence that directs transcription of DNA into RNA.

Protein S activity - any property of human protein S responsible for biological function or anti-human protein S antibody-binding activity.

Replicon - A DNA sequence that controls and allows for autonomous replication of a plasmid or other vector.

Structural gene - any DNA sequence that encodes a functional polypeptide, inclusive of translational start and stop signals.

TcR - the tetracycline-resistant phenotype or gene conferring same.

Translational Activating Sequence - any DNA sequence, inclusive of that encoding a ribosome binding site and translational start codon, such as 5'-ATG-3', that provides for the translation of a mRNA transcript into a peptide or polypeptide.

The present invention relates to novel DNA compounds and recombinant DNA expression vectors that encode a polypeptide precursor for human protein S activity. The vectors allow expression of the novel DNA compounds in eukaryotic host cells. The invention also relates to host cells transformed with these vectors. Human liver cDNA coding for protein S has been cloned and sequenced. The cDNA sequence consists of a coding region for a 676 amino acid precursor, flanked by 5'- and 3'-noncoding segments. The coding region encodes a 41 amino acid leader peptide, 635 amino acids corresponding to the mature protein and a stop codon. Accordingly, it is an object of the present invention to provide the DNA sequence coding for human protein S and its precursor.

It is a further object of the present invention to provide the primary structure of the protein possessing human protein S activity.

Additionally, it is an object of the present invention to provide a plasmid comprising a gene encoding human protein S precursor.

Brief Description of the Drawings

Figure 1 is a restriction site and function map of plasmid pShd.

Figure 2 is a restriction site and function map of plasmid pHHS-IIa. The Pstl restriction site noted in parenthesis is not reconstituted.

Figure 3 is a restriction site and function map of plasmid pBKE1.

Figure 4 is a restriction site and function map of plasmid pBkneo1.

Figure 5 is a restriction site and function map of plasmid pSV2cat.

Figure 6 is a restriction site and function map of plasmid pLPcat.

Figure 7 is a restriction site and function map of plasmid pBLcat.

Figure 8 is a flow chart illustrating the construction of plasmid pL133.

Figure 9 is a restriction site and function map of plasmid pLPC.

Figure 10 is a restriction site and function map of plasmid pLPC4.

Figure 11 is a restriction site and function map of plasmid pSV2hyg.

Figure 12 is a restriction site and function map of plasmid pLPChyg1.

Figure 13 is a restriction site and function map of plasmid pLPChd1.

Figure 14 is a restriction site and function map of plasmid phd. The present invention relates to recombinant human protein S produced by means of a double-stranded deoxyribonucleic acid that encodes a polypeptide with human protein S activity. The entire amino acid sequence of human protein S precursor, as well as the coding strand of DNA encoding a polypeptide with human protein S activity is presented below.

```
                    10              20              30              40
ATG AGG GTC CTG GGT GGG CGC TGC GGG GCG CCG CTG GCG TGT
MET ARG VAL LEU GLY GLY ARG CYS GLY ALA PRO LEU ALA CYS
                     5                              10

                    50              60              70              80
CTC CTC CTA GTG CTT CCC GTC TCA GAG GCA AAC CTT CTG TCA
LEU LEU LEU VAL LEU PRO VAL SER GLU ALA ASN LEU LEU SER
 15                             20                      25

           90              100             110             120
AAG CAA CAG GCT TCA CAA GTC CTG GTT AGG AAG CGT CGT GCA
LYS GLN GLN ALA SER GLN VAL LEU VAL ARG LYS ARG ARG ALA
      30                      35                      40

          130             140             150             160
AAT TCT TTA CTT GAA GAA ACC AAA CAG GGT AAT CTT GAA AGA
ASN SER LEU LEU GLU GLU THR LYS GLN GLY ASN LEU GLU ARG
             45                      50                      55

    170             180             190             200             210
GAA TGC ATC GAA GAA CTG TGC AAT AAA GAA GAA GCC AGG GAG
GLU CYS ILE GLU GLU LEU CYS ASN LYS GLU GLU ALA ARG GLU
                     60                      65                      70

          220             230             240             250
GTC TTT GAA AAT GAC CCG GAA ACG GAT TAT TTT TAT CCA AAA
VAL PHE GLU ASN ASP PRO GLU THR ASP TYR PHE TYR PRO LYS
                     75                      80
```

5

```
              260           270           280           290
TAC TTA GTT TGT CTT CGC TCT TTT CAA ACT GGG TTA TTC ACT
TYR LEU VAL CYS LEU ARG SER PHE GLN THR GLY LEU PHE THR
 85                        90                        95
              300           310           320           330
GCT GCA CGT CAG TCA ACT AAT GCT TAT CCT GAC CTA AGA AGC
ALA ALA ARG GLN SER THR ASN ALA TYR PRO ASP LEU ARG SER
         100                        105                        110
              340           350           360           370
TGT GTC AAT GCC ATT CCA GAC CAG TGT AGT CCT CTG CCA TGC
CYS VAL ASN ALA ILE PRO ASP GLN CYS SER PRO LEU PRO CYS
                  115                        120                        125
     380           390           400           410           420
AAT GAA GAT GGA TAT ATG AGC TGC AAA GAT GGA AAA GCT TCT
ASN GLU ASP GLY TYR MET SER CYS LYS ASP GLY LYS ALA SER
                        130                   135                        140
              430           440           450           460
TTT ACT TGC ACT TGT AAA CCA GGT TGG CAA GGA GAA AAG TGT
PHE THR CYS THR CYS LYS PRO GLY TRP GLN GLY GLU LYS CYS
                       145                   150
          470           480           490           500
GAA TTT GAC ATA AAT GAA TGC AAA GAT CCC TCA AAT ATA AAT
GLU PHE ASP ILE ASN GLU CYS LYS ASP PRO SER ASN ILE ASN
155                        160                        165
          510           520           530           540
GGA GGT TGC AGT CAA ATT TGT GAT AAT ACA CCT GGA AGT TAC
GLY GLY CYS SER GLN ILE CYS ASP ASN THR PRO GLY SER TYR
         170                        175                        180
          550           560           570           580
CAC TGT TCC TGT AAA AAT GGT TTT GTT ATG CTT TCA AAT AAG
HIS CYS SER CYS LYS ASN GLY PHE VAL MET LEU SER ASN LYS
             185                        190                        195
```

```
         590                600                610                620                630
AAA GAT TGT AAA GAT GTG GAT GAA TGC TCT TTG AAG CCA AGC
LYS ASP CYS LYS ASP VAL ASP GLU CYS SER LEU LYS PRO SER
                 200                205                210

         640                650                660                670
ATT TGT GGC ACA GCT GTG TGC AAG AAC ATC CCA GGA GAT TTT
ILE CYS GLY THR ALA VAL CYS LYS ASN ILE PRO GLY ASP PHE
                 215                220

         680                690                700                710
GAA TGT GAA TGC CCC GAA GGC TAC AGA TAT AAT CTC AAA TCA
GLU CYS GLU CYS PRO GLU GLY TYR ARG TYR ASN LEU LYS SER
225                230                235

         720                730                740                750
AAG TCT TGT GAA GAT ATA GAT GAA TGC TCT GAG AAC ATG TGT
LYS SER CYS GLU ASP ILE ASP GLU CYS SER GLU ASN MET CYS
         240                245                250

         760                770                780                790
GCT CAG CTT TGT GTC AAT TAC CCT GGA GGT TAC ACT TGC TAT
ALA GLN LEU CYS VAL ASN TYR PRO GLY GLY TYR THR CYS TYR
                 255                260                265

         800                810                820                830                840
TGT GAT GGG AAG AAA GGA TTC AAA CTT GCC CAA GAT CAG AAG
CYS ASP GLY LYS LYS GLY PHE LYS LEU ALA GLN ASP GLN LYS
                 270                275                280

         850                860                870                880
AGT TGT GAG GTT GTT TCA GTG TGC CTT CCC TTG AAC CTT GAC
SER CYS GLU VAL VAL SER VAL CYS LEU PRO LEU ASN LEU ASP
                 285                290

         890                900                910                920
ACA AAG TAT GAA TTA CTT TAC TTG GCG GAG CAG TTT GCA GGG
THR LYS TYR GLU LEU LEU TYR LEU ALA GLU GLN PHE ALA GLY
295                300                305
```

```
          930              940              950              960
GTT GTT TTA TAT TTA AAA TTT CGT TTG CCA GAA ATC AGC AGA
VAL VAL LEU TYR LEU LYS PHE ARG LEU PRO GLU ILE SER ARG
         310               315                 320
          970         980          990             1000
TTT TCA GCA GAA TTT GAT TTC CGG ACA TAT GAT TCA GAA GGC
PHE SER ALA GLU PHE ASP PHE ARG THR TYR ASP SER GLU GLY
         325               330                 335
   1010          1020          1030          1040          1050
GTG ATA CTG TAC GCA GAA TCT ATC GAT CAC TCA GCG TGG CTC
VAL ILE LEU TYR ALA GLU SER ILE ASP HIS SER ALA TRP LEU
              340               345                 350
          1060          1070          1080          1090
CTG ATT GCA CTT CGT GGT GGA AAG ATT GAA GTT CAG CTT AAG
LEU ILE ALA LEU ARG GLY GLY LYS ILE GLU VAL GLN LEU LYS
              355               360
        1100          1110          1120          1130
AAT GAA CAT ACA TCC AAA ATC ACA ACT GGA GGT GAT GTT ATT
ASN GLU HIS THR SER LYS ILE THR THR GLY GLY ASP VAL ILE
365               370                 375
      1140          1150          1160          1170
AAT AAT GGT CTA TGG AAT ATG GTG TCT GTG GAA GAA TTA GAA
ASN ASN GLY LEU TRP ASN MET VAL SER VAL GLU GLU LEU GLU
     380               385                 390
      1180          1190          1200          1210
CAT AGT ATT AGC ATT AAA ATA GCT AAA GAA GCT GTG ATG GAT
HIS SER ILE SER ILE LYS ILE ALA LYS GLU ALA VAL MET ASP
          395               400                 405
   1220          1230          1240          1250          1260
ATA AAT AAA CCT GGA CCC CTT TTT AAG CCG GAA AAT GGA TTG
ILE ASN LYS PRO GLY PRO LEU PHE LYS PRO GLU ASN GLY LEU
         410               415                 420
```

```
          1270            1280            1290            1300
CTG GAA ACC AAA GTA TAC TTT GCA GGA TTC CCT CGG AAA GTG
LEU GLU THR LYS VAL TYR PHE ALA GLY PHE PRO ARG LYS VAL
                    425                     430
           1310            1320            1330            1340
GAA AGT GAA CTC ATT AAA CCG ATT AAC CCT CGT CTA GAT GGA
GLU SER GLU LEU ILE LYS PRO ILE ASN PRO ARG LEU ASP GLY
435                     440                     445
           1350            1360            1370            1380
TGT ATA CGA AGC TGG AAT TTG ATG AAG CAA GGA GCT TCT GGA
CYS ILE ARG SER TRP ASN LEU MET LYS GLN GLY ALA SER GLY
           450                     455                     460
           1390            1400            1410            1420
ATA AAG GAA ATT ATT CAA GAA AAA CAA AAT AAG CAT TGC CTG
ILE LYS GLU ILE ILE GLN GLU LYS GLN ASN LYS HIS CYS LEU
                    465                     470                     475
   1430            1440            1450            1460            1470
GTT ACT GTG GAG AAG GGC TCC TAC TAT CCT GGT TCT GGA ATT
VAL THR VAL GLU LYS GLY SER TYR TYR PRO GLY SER GLY ILE
                    480                     485                     490
           1480            1490            1500            1510
GCT CAA TTT CAC ATA GAT TAT AAT AAT GTA TCC AGT GCT GAG
ALA GLN PHE HIS ILE ASP TYR ASN ASN VAL SER SER ALA GLU
                    495                     500
           1520            1530            1540            1550
GGT TGG CAT GTA AAT GTG ACC TTG AAT ATT CGT CCA TCC ACG
GLY TRP HIS VAL ASN VAL THR LEU ASN ILE ARG PRO SER ·THR
505                     510                     515
           1560            1570            1580            1590
GGC ACT GGT GTT ATG CTT GCC TTG GTT TCT GGT AAC AAC ACA
GLY THR GLY VAL MET LEU ALA LEU VAL SER GLY ASN ASN THR
           520                     525                     530
```

9

```
       1600            1610            1620            1630
GTG CCC TTT GCT GTG TCC TTG GTG GAC TCC ACC TCT GAA AAA
VAL PRO PHE ALA VAL SER LEU VAL ASP SER THR SER GLU LYS
            535                     540                     545
    1640            1650            1660            1670            1680
TCA CAG GAT ATT CTG TTA TCT GTT GAA AAT ACT GTA ATA TAT
SER GLN ASP ILE LEU LEU SER VAL GLU ASN THR VAL ILE TYR
                550                     .555                    560
                1690            1700            1710            1720
CGG ATA CAG GCC CTA AGT CTA TGT TCC GAT CAA CAA TCT CAT
ARG ILE GLN ALA LEU SER LEU CYS SER ASP GLN GLN SER HIS
                    565                     570
            1730            1740            1750            1760
CTG GAA TTT AGA GTC AAC AGA AAC AAT CTG GAG TTG TCG ACA
LEU GLU PHE ARG VAL ASN ARG ASN ASN LEU GLU LEU SER THR
575                     580                     585
        1770            1780            1790            1800
CCA CTT AAA ATA GAA ACC ATC TCC CAT GAA GAC CTT CAA AGA
PRO LEU LYS ILE GLU THR ILE SER HIS GLU ASP LEU GLN ARG
        590                     595                     600
    1810            1820            1830            1840
CAA CTT GCC GTC TTG GAC AAA GCA ATG AAA GCA AAA GTG GCC
GLN LEU ALA VAL LEU ASP LYS ALA MET LYS ALA LYS VAL ALA
            605                     610                     615
    1850            1860            1870            1880            1890
ACA TAC CTG GGT GGC CTT CCA GAT GTT CCA TTC AGT GCC ACA
THR TYR LEU GLY GLY LEU PRO ASP VAL PRO PHE SER ALA THR
                620                     625                     630
            1900            1910            1920            1930
CCA GTG AAT GCC TTT TAT AAT GGC TGC ATG GAA GTG AAT ATT
PRO VAL ASN ALA PHE TYR ASN GLY CYS MET GLU VAL ASN ILE
                635                     640
```

```
        1940              1950              1960              1970
AAT GGT GTA CAG TTG GAT CTG GAT GAA GCC ATT TCT AAA CAT
ASN GLY VAL GLN LEU ASP LEU ASP GLU ALA ILE SER LYS HIS
645                     650                     655
        1980              1990              2000              2010
AAT GAT ATT AGA GCT CAC TCA TGT CCA TCA GTT TGG AAA AAG
ASN ASP ILE ARG ALA HIS SER CYS PRO SER VAL TRP LYS LYS
        660                     665                     670
2020
ACA AAG AAT TCT
THR LYS ASN SER
        675
```

The amino acid sequence of the protein S precursor is based upon the cDNA sequence. The precursor schematically consists of seven distinct domains and one unknown region. These regions are grouped by approximate amino acid (aa) boundries as follows: leader peptide (aa-41 to -1); a $\gamma$-carboxyglutamate (Gla) segment (aa 1-37); a linker region (aa 38-73); four epidermal growth factor domains (aa 74-121, 122-165, 166-207, 208-248); and an unknown region (aa 249-635). The numbering of amino acid residues herein is based upon the assignment of +1 to the amino terminal residue of the mature protein (residue number 42 in the precursor protein sequence shown above). The invention further provides recombinant DNA plasmids comprising a gene encoding human protein S and micro-organisms or cell lines transformed by these plasmids.

The DNA compounds of the present invention are derived from a single cDNA clone prepared from human liver mRNA that encodes the entire gene, including both 5' and 3' noncoding sequences, for human protein S precursor

Initially, a cDNA library was prepared in a lambda ($\lambda$) gtll expression vector (lac5 nin5 cl857 S100) according to the procedure described by Young and Davis, 1983, Proc. Natl. Acad. Sci. USA 80:1194-1198.

The recombinant human liver cDNA library constructed in $\lambda$gt11 was obtained from Clontech Laboratories, Inc., 922 Industrial Avenue, Palo Alto, California 94303. This library was screened using conventional methods with polyclonal antibodies raised in a goat against human protein S. Approximately 2 x 10[6] individual recombinants were screened in the form of phage plaques on a lawn of Escherichia coli Y1090. E. coli Y1090 is available from the ATCC under the accession number 37197.

To protect against the possibility that the fusion protein might be toxic to the host, plaque formation was initiated without expression from the lacZ gene promoter by incubating the plates at 42°C. After the number of infected cells surrounding the plaques was large, lacZ-directed gene expression was induced by the addition of isopropylthio-$\beta$-galactoside (IPTG). This was accomplished by placing an IPTG-impregnated nitrocellulose filter disk over the lawn. After a period of incubation, the nitrocellulose filters with bound protein were washed, treated with primary antibody, then biotinylated secondary antibody, followed by avidin-peroxidase conjugate. Positive colonies were identified by a color reaction when exposed to hydrogen peroxide.

The identified positives were picked and re-tested at a density of about 5000 plaques per plate. Several of the positive plaques were picked and re-screened at a density of about 400 plaques per plate. Using isolated plaques from the latter screen, large amounts of purified phage DNA were isolated.

The initial step taken to map the cDNA insert in the phage clones was to determine the size(s) of the EcoRI fragment(s). In the construction of the library, the cDNA fragments were ligated to artificial EcoRI linkers and inserted into a unique EcoRI site in the $\lambda$gt11 molecule. Digesting a recombinant phage DNA molecule with EcoRI would generate the two lambda "arms" (19.6 and 24.1 Kb); any other fragments produced thereby correspond to the inserted DNA. (If the DNA sequence of the insert contained no internal EcoRI site then only one EcoRI fragment would be generated. Correspondingly, one or more internal EcoRI sites would result in two or more additional EcoRI fragments). Accordingly, the purified phage DNA was digested with EcoRI and separated by electrophoresis on either a 3.5% polyacrylamide gel or a 1.0% agarose gel. One of the largest clones, designated 1-1, contained two EcoRI fragments of 1.8 Kb and 0.8 Kb. The EcoRI fragments isolated from this clone were subsequently subcloned into plasmid pBR322 and used to transform E. coli RR1 cells.

After transformation and plasmid identification, the plasmid DNA was amplified and purified in preparation for DNA sequencing. The subcloned DNA fragments from clones designated pLHS-21 and pLHS-22 were sequenced according to the teaching of Maxam and Gilbert, 1980, Methods in Enzymology 61:497.

Localization of the particular DNA sequences within the EcoRI-generated restriction fragments was accomplished by Southern transfer analysis. Two independent, mixed probes, described below in Table 1, were used for the hybridization procedure. These probes were chemically synthesized either by using a Systec 1450A DNA Synthesizer (Systec Inc., 3816 Chandler Drive, Minneapolis, MN 55421) or an ABS 380A DNA Synthesizer (Applied Biosystems, Inc., 850 Lincoln Centre Drive, Foster City, CA 94404). Many DNA synthesizing instruments are known in the art and can be used to make the fragments. In addition, the fragments can also be conventionally prepared in substantial accordance with the procedures of Itakura et al., 1977, Science 198:1056 and Crea et al., 1978, Proc. Natl. Acad. Sci. USA 75:5765.

## Table 1

---

**Probe 80:**                                23mer/ 128 combinations

$$3'--AC^A_GTT^A_GCT^T_CCT^A_GCCNAA^A_GTACTG--5'$$

**Probe 81:**                                20mer/ 64 combinations

$$3'--AACGT^T_CTT^T_CTGNTT^T_CGT^A_GTG--5'$$

---

Probe 80 corresponds to amino acid residues 83-90 of one of the EGF domains of the bovine amino acid sequence presented by J. Stenflo (Xth International Congress on Thrombosis and Haemostasis, San Diego, California, July, 1985). Probe 81 was also based upon the bovine sequence and corresponds to the carboxy terminal amino acid residues 628-634. These two probes hybridized strongly to the ~1.8 Kb EcoRI subcloned fragment.

Once the sequencing data had been obtained, it became apparent that the complete coding sequence of protein S, specifically the 5' end of the molecule, was not represented in the clones generated from the λgt11 library. Therefore, a second library was utilized to generate the missing sequence. Based upon the sequenced DNA, two probes, designated 21 and 22 in Table 2 below, were derived from identified λgt11 clones.

12

## Table 2

---

### Probe 22

#### HinfI

GAATCTTCTT CTTGGCAGCT GCAGTCTGTC AGGATGAGAT ATCAGATTAG

GTTGGATAGG TGGGGAAATC TGAAGTGGGT ACATTTTTTA AATTTTGCTG

                                                         EcoRI

TGTGGGTCAC ACAAGGTCTA CATTACAAAA GACAGAATTC

### Probe 21

#### HincII

GTCAACTAAT GCTTATCCTG ACCTAAGAAG CTGTGTCAAT GCCATTCCAG

ACCAGTGTAG TCCTCTGCCA TGCAATGAAG ATGGATATAT GAGCTGCAAA

              HindIII

GATGGAAAAG CTT

---

Probe 22 corresponds to a 3' non-translated region of the molecule and was used to locate 3' regions of cDNA. In contrast, probe 21, corresponding to a region of the 5' end of the molecule, was used to locate the 5' coding portion of the molecule. These probes were radioactively labelled and used to probe the cDNA library. After hybridization, several positives were identified using restriction enzyme mapping, Southern hybridization and DNA sequencing. One clone, designated pHHS-IIa contains sequences corresponding to the complete protein S precursor as well as 5' and 3' non-coding segments.

Plasmid pHHS-IIa contains ~3.3 Kb of cDNA corresponding to messenger RNA (mRNA) which is significantly larger than the ~2.5 Kb size reported by Stenflo, 1985. Northern analysis of both bovine and human liver mRNA with human cDNA probes shows that the RNA species differ in a manner consistent with the sizes of the cDNAs.

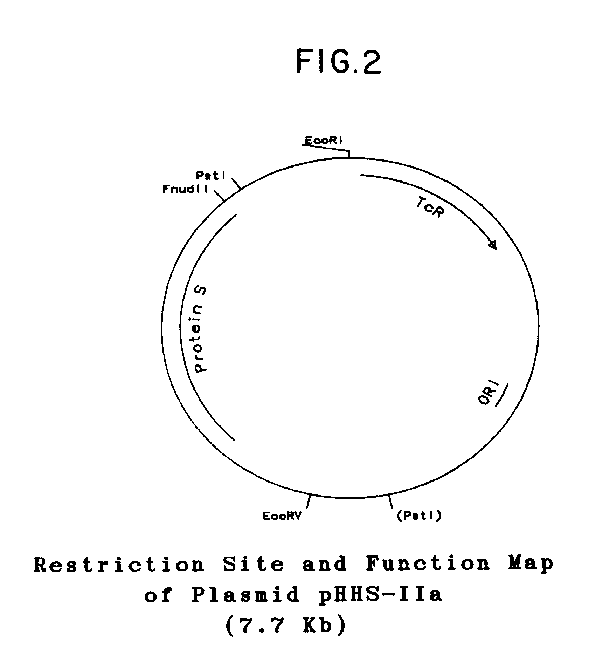

Plasmid pHHS-IIa can be conventionally isolated from E. coli K12RRI/pHHS-IIa, a strain deposited with and made part of the permanent stock culture collection of the Northern Regional Research Laboratory (NRRL), Peoria, Illinois. A culture of E. coli K12 RR1/pHHS-IIa can be obtained from the NRRL under the accession number B-18071. A restriction site and function map of plasmid pHHS-IIa is presented in Figure 2 of the accompanying drawings.

Alternatively, the specific DNA sequences of the present invention can be chemically synthesized by using a DNA synthesizer as previously described. Despite the rather large size of the protein S gene, double-stranded DNA segments containing more than 1000 deoxynucleotides are generated quite easily today with the aid of these "gene machines" (see Caruthers, M. H., 1985, Science 230:281).

A variety of recombinant DNA expression vectors comprising the protein S activity-encoding DNA can be constructed. Many mammalian host cells possess the necessary cellular machinery for the recognition and proper processing of the leader peptide present on the amino-terminus of human protein S. Some mammalian host cells also provide the post-translational modifications, such as glycosylation, $\gamma$-carboxylation, and $\beta$-hydroxylation, as are observed in human protein S present in blood plasma. A wide variety of vectors exist for the transformation of eukaryotic host cells, and the specific vectors exemplified below are in no way intended to limit the scope of the present invention.

Two embodiments of the present invention utilize pSV2-type vectors as starting material in the construction of eukaryotic expression vectors. The pSV2-type vectors comprise segments of the SV40 genome that constitute a defined eukaryotic transcription unit-promoter (ep), intervening sequence (IVS), and polyadenylation (pA) site. In the absence of SV40 T-antigen, the plasmid pSV2-type vectors transform mammalian and other eukaryotic host cells by integrating into the host cell chromosomal DNA. A variety of plasmid pSV2-type vectors are known in the art (see Eukaryotic Viral Vectors, edited by Gluzman, published by Cold Spring Harbor Laboratories, Cold Spring Harbor, New York, 1982), such as plasmids pSV2-gpt, pSV2-neo, pSV2-dhfr, and pSV2-β-globin, in which the SV40 promoter drives transcription of an inserted gene. These vectors are available either from the American Type Culture Collection (ATCC) in Rockville, Maryland or from the aforementioned NRRL.

Amino acid residues -41 to -18 at the amino-terminus of human protein S may act as a signal peptide to direct secretion of protein S from the liver into the bloodstream. These residues are not present in mature protein S. Residues -17 to -1 of human protein S precursor, which likely comprise the pro-peptide of human protein S, are also removed during the processing and activation of the protein and are believed responsible for the correct folding and modification of the molecule. Although not specifically exemplified herein, the present invention also comprises protein S derivatives such as "pro-protein S". The present DNA compounds are readily modified to delete that portion. encoding amino and residues -41 to -18 of human protein S precursor for expression of the resulting derivative.

Skilled artisans will recognize that the present invention is not limited to the expression of a particular protein S derivation in eukaryotic hosts. As a general rule, prokaryotes do not efficiently process eukaryotic signal peptides; therefore, it would be somewhat inefficient to express the signal peptide-encoding portion of the human protein S precursor gene in prokaryotes. Although not specifically exemplified herein, the present invention also comprises the fusion of a prokaryotic signal peptide-encoding DNA to the protein S activity-encoding DNA of the present invention for expression and secretion of protein S activity in prokaryotes.

Useful starting materials for the preparation of the expression vectors of the present invention include expression vectorsin which the BK virus enhancer (Enh) is used in the presence of an immediate-early gene product of a large DNA virus to increase transcription of a recombinant gene in eukaryotic host cells. Thus, such vectors are useful to express the protein S encoding gene of the present invention. The construction of these vectors is described in detail in Examples 8-17. A brief over-view of their construction immediately follows.

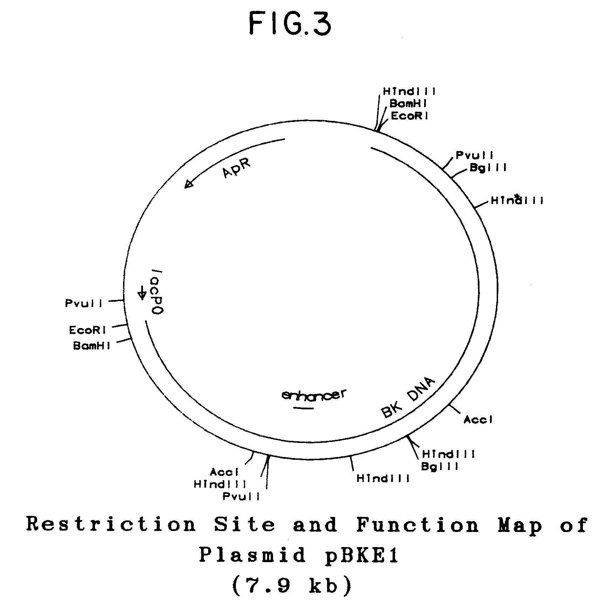

The BK virus (ATCC VR-837) can be purchased or readily isolated in large quantities as described in Example 8. Alternatively, it is also convenient to clone the BK viral DNA onto a plasmid cloning vector and use the recombinant vector as a source of BK viral DNA sequences. Consequently, BK viral DNA was digested with restriction enzyme EcoRI, which, due to the presence of only one EcoRI site on the BK genome, produced linear BK DNA. Plasmid pUC8 (available from Bethesda Research Laboratories (BRL), P.O. Box 6009, Gaithersburg, MD 20877) was likewise digested and linearized with restriction enzyme EcoRI, and the EcoRI-cut plasmid pUC8 DNA was ligated to the EcoRI-cut BK viral DNA to form plasmids pBKE1 and pBKE2, which differ only with respect to the orientation of the BK viral DNA. A restriction site and function map of plasmid pBKE1 is presented in Figure 3 of the accompanying drawings. The construction of plasmids pBKE1 and pBKE2 is described in Example 9.

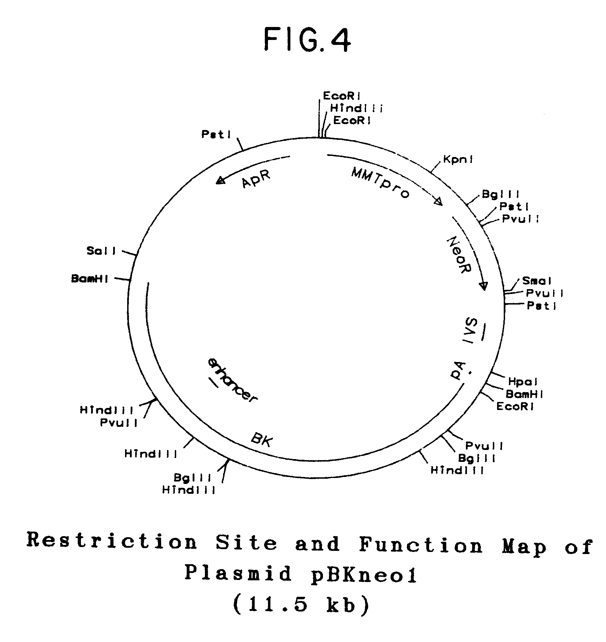

The BK viral genome has also been combined with a portion of plasmid pdBPV-MMTneo to construct plasmids pBKneo1 and pBKneo2. Plasmid pdBPV-MMTneo, about 15 Kb in size and available from the ATCC under the accession number ATCC 37224, comprises the replicon and β-lactamase gene from plasmid pBR322, the mouse metallothionein promoter (MMTpro) positioned to drive expression of a structural gene that encodes a neomycin resistance-conferring enzyme, and about 8 Kb of bovine papilloma virus (BPV) DNA. Plasmid pdBPV-MMTneo can be digested with restriction enzyme BamHI to generate two fragments: the ~8 Kb fragment that comprises the BPV DNA and an ~7 Kb fragment that comprises the other sequences described above. BK virus has only one BamHI restriction site, and plasmids pBKneo1 and pBKneo2 were constructed by ligating the ~7 Kb BamHI restriction fragment of plasmid pdBPV-MMTneo to BamHI-linearized BK virus DNA. The construction of plasmids pBKneo1 and pBKneo2, which differ only with respect to the orientation of the BK virus DNA, is described in Example 10, and a restriction site and function map of plasmid pBKneo1 is presented in Figure 4 of the accompanying drawings.

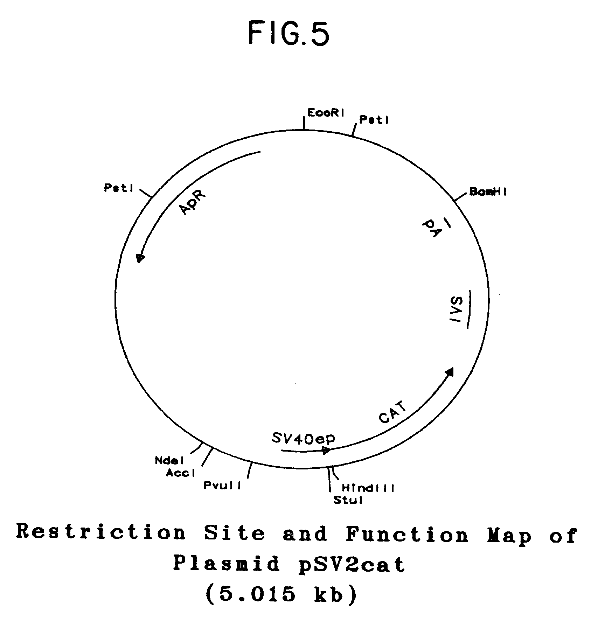

An additional vector, useful as starting material in the construction of the present expression vectors, is designated plasmid pBLcat. This plasmid comprises the BK enhancer sequence in tandem with the human adenovirus-type-2 late promoter (Ad2LP) positioned to drive expression of the chloramphenicol acetyltransferase enzyme (CAT). Plasmid pSV2cat serves as a convenient source of the CAT gene and can be obtained from the ATCC under the accession number ATCC 37155. A restriction site and function map of plasmid pSV2cat is presented in Figure 5 of the accompanying drawings. Human adenovirus-type-2 DNA is commercially available and can also be obtained from the ATCC under the accession number ATCC VR-2.

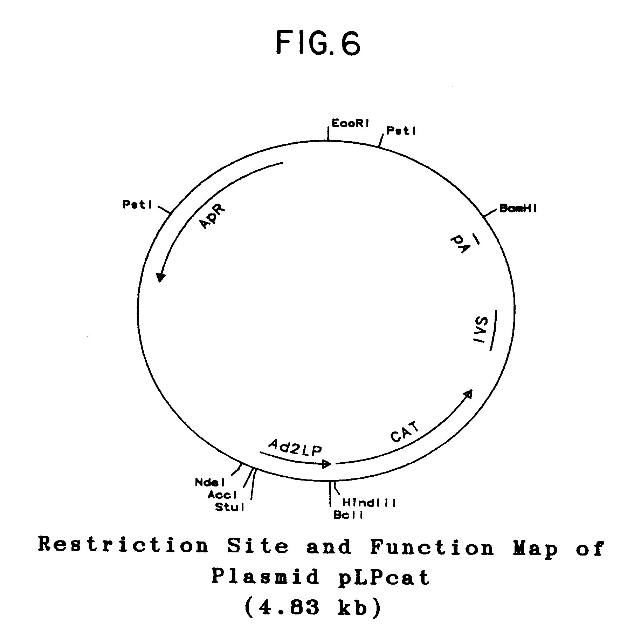

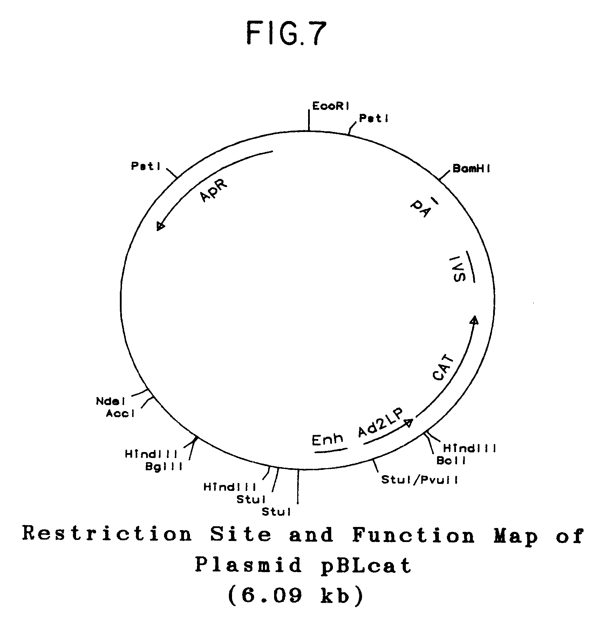

Briefly, plasmid pBLcat was constructed by ligating the ~0.32 Kb late-promoter-containing AccI-PvuII restriction fragment of human adenovirus-type-2 DNA to blunt-ended BclI linkers that attached only to the PvuII end of the AccI-PvuII restriction fragment. The resulting fragment was then ligated to the ~4.51 Kb AccI-StuI restriction fragment of plasmid pSV2cat to yield an intermediate plasmid pLPcat, for which a restriction site and function map is presented in Figure 6 of the accompanying drawings. The desired plasmid pBLcat was constructed from plasmid pLPcat by ligating the origin of replication and enhancer-containing, ~1.28 Kb AccI-PvuII restriction fragment of BK virus DNA to the ~4.81 Kb AccI-StuI restriction fragment of plasmid pLPcat. A restriction site and function map of the resultant plasmid pBLcat is presented in Figure 7 of the accompanying drawings. The construction of plasmid pBLcat is further described in Example 11.

Plasmid pBLcat is a convenient source of a BK enhancer-adenovirus late promoter "cassette" which is an ~870 bp HindIII restriction fragment that can be conveniently inserted into a eukaryotic expression vector to increase expression of a product encoded by that vector.

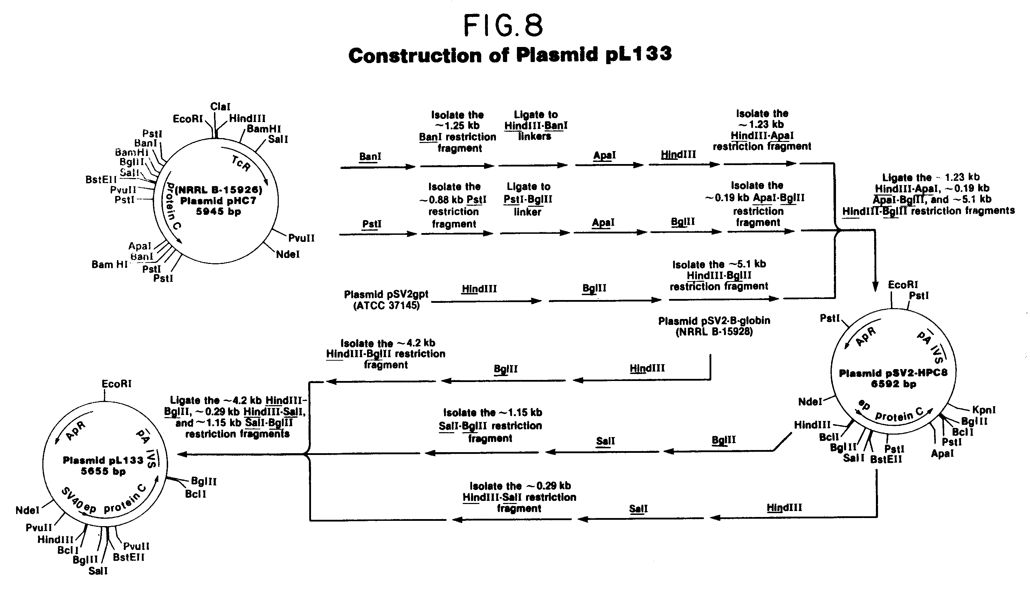

For example, the BK enhancer-adenovirus late promoter cassette was used to improve expression of human protein C by ligating the cassette into plasmid pL133. A restriction site and function map of plasmid pL133 is presented in Figure 8 of the accompanying drawings.

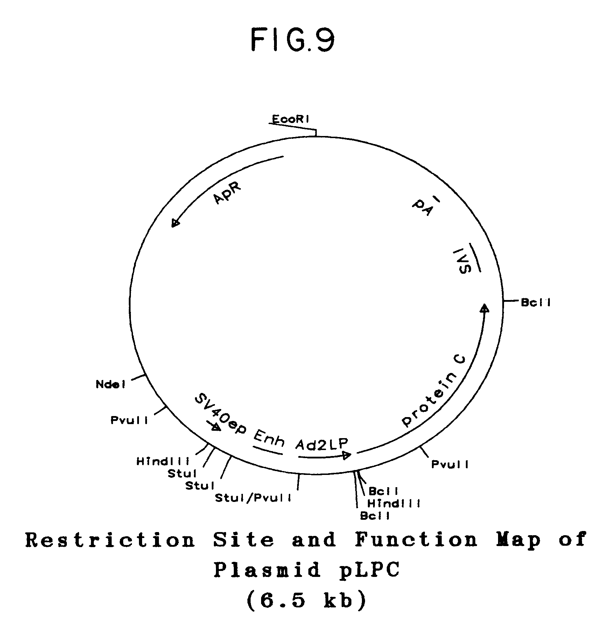

Plasmid pL133 is also a convenient expression vector to use for the expression of protein S. This plasmid can be digested with restriction enzyme BclI (to delete the fragment containing the protein C gene) and treated with Klenow to generate a linear, blunt-ended fragment. The entire protein S gene, contained on an ~2.7 Kb FnuDII-EcoRV restriction fragment can then be ligated to the Klenow-treated BclI vector fragment to generate a protein S expression vector. Plasmid pL133, the construction of which is given in Example 12, is also used as an intermediate vector to construct additional expression vectors. Thus, the plasmid was digested with restriction enzyme HindIII and then ligated to the ~0.87 Kb HindIII restriction fragment of plasmid pBLcat to yield plasmid pLPC. A restriction site and function map of plasmid pLPC is presented in Figure 9 of the accompanying drawings, and the construction of plasmid pLPC is further described in Example 13.

Plasmid pLPC, like plasmid pL133, comprises the enhancer, early and late promoters, T-antigen-binding sites, and origin of replication of SV40. These elements are closely situated together on the SV40 DNA and are difficult to delineate. The binding of T antigen to the T-antigen-binding sites, which is necessary for SV40 replication, is known to enhance transcription from the SV40 late promoter and surprisingly has a similar effect on the BK early and late promoters. Because the high level of T-antigen-driven replication of a plasmid that comprises the SV40 origin of replication is generally lethal to the host cell, neither plasmid pLPC nor plasmid pL133 are stably maintained as episomal (extrachromosomal) elements in the presence of SV40 T antigen, but rather, the two plasmids must integrate into the chromosomal DNA of the host cell to be stably maintained.

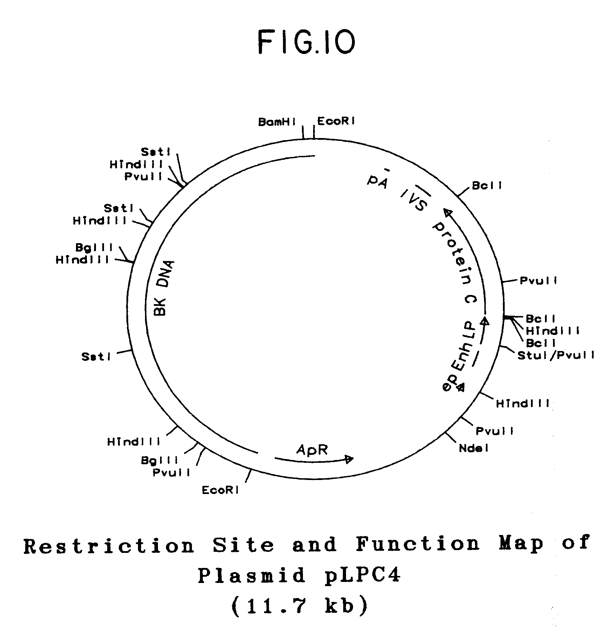

To construct a derivative of plasmid pLPC that can exist as a stably-maintained element in a transformed eukaryotic cell, the entire BK genome, as an EcoRI-linerarized restriction fragment, was inserted into the single EcoRI restriction site of plasmid pLPC. This insertion produced two plasmids, designated pLPC4 and pLPC5, that differ only with respect to the orientation of the BK EcoRI fragment. A restriction site and function map of plasmid pLPC4 is presented in Figure 10 of the accompanying drawings, and the construction of plasmids pLPC4 and pLPC5 is further described in Example 14.

Episomal maintenance of a recombinant DNA expression vector is not always preferred over integration into the host cell chromosome. However, due to the absence of a selectable marker that functions in eukaryotic cells, the identification of stable, eukaryotic transformants of plasmid pLPC is difficult, unless plasmid pLPC is cotransformed with another plasmid that does comprise a selectable marker. Consequently, plasmid pLPC has been modified to produce derivative plasmids that are selectable in eukaryotic host cells.

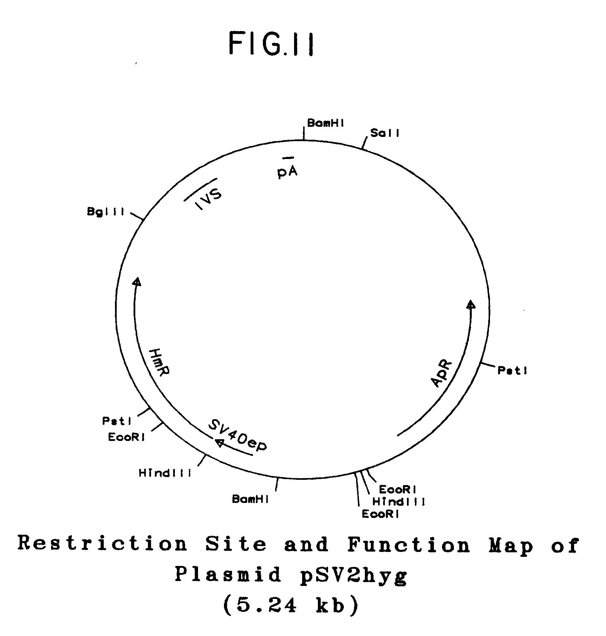

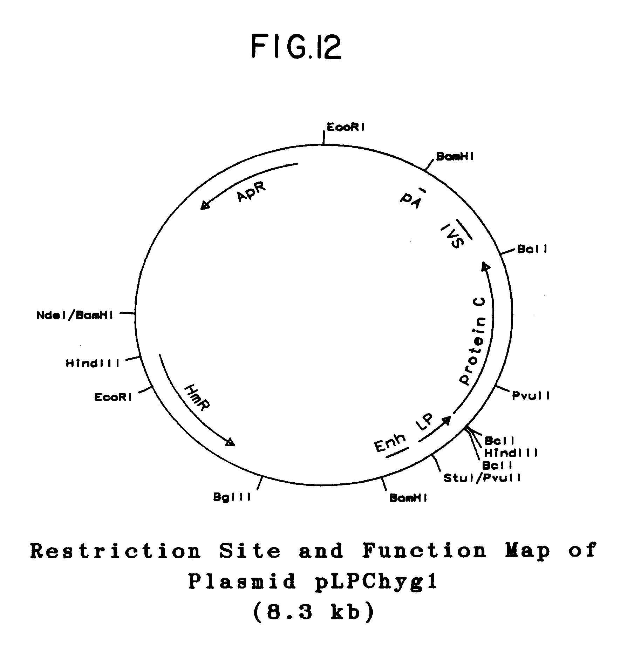

This was done by ligating plasmid pLPC to a portion of plasmid pSV2hyg, a plasmid that comprises a hygromycin resistance-conferring gene. A restriction site and function map of plasmid pSV2hyg, which can be obtained from the NRRL, under the accession number NRRL B-18039, is presented in Figure 11 of the accompanying drawings. Plasmid pSV2hyg was digested with restriction enzyme BamHI, and the ~2.5 Kb BamHI restriction fragment, which comprises the entire hygromycin resistance-conferring gene, was isolated, treated with Klenow enzyme (the large fragment produced upon subtilisin cleavage of E. coli DNA polymerase I), and then ligated to the Klenow-treated, ~5.82 Kb NdeI-StuI restriction fragment of plasmid pLPC to yield plasmids pLPChyg1 and pLPChyg2. Plasmids pLPChyg1 and pLPChyg2 differ only with respect to the orientation of the hygromycin resistance-conferring fragment. A restriction site and function map of plasmid pLPChyg1 is presented in Figure 12 of the accompanying drawings, and the construction protocol for plasmids pLPChygl and pSPChyg2 is described in Example 15.

Plasmid pLPChyg1 was modified to introduce a dihydrofolate reductase (dhfr) gene. The dhfr gene is a selectable marker in dhfr-negative cells and can be used to increase the copy number of a DNA segment by exposing the host cell to increasing levels of methotrexate. The dhfr gene was obtained from plasmid pSV2-dhfr (ATCC 37146). The use of a particular dhfr gene is not critical in the construction of the present

EP 0 247 843 B1

vectors.

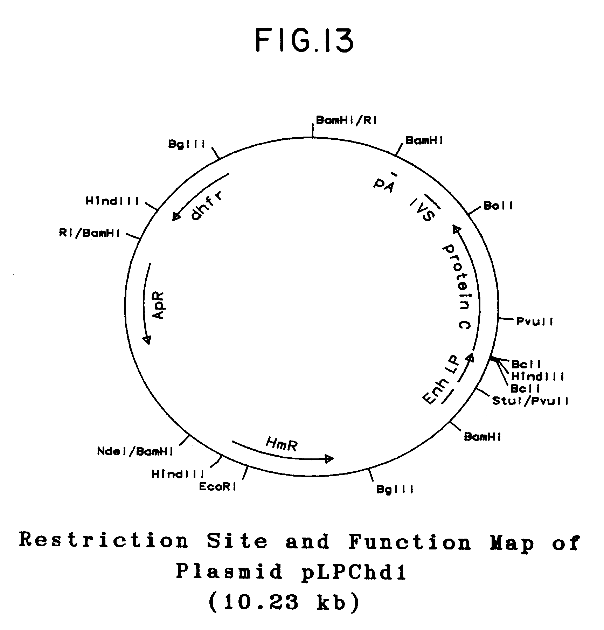

After isolation, the dhfr gene-containing, ~1.9 Kb BamHI restriction fragment was treated with Klenow enzyme and inserted into partially-EcoRI-digested plasmid pLPChyg1 to yield plasmids pLPChd1 and pLPChd2. Plasmid pSPChyg1 contains two EcoRI restriction enzyme recognition sites, one in the hygromycin resistance-conferring gene and one in the plasmid pBR322-derived sequences. The fragment comprising the dhfr gene was inserted into the EcoRI site located in the pBR322-derived sequences of plasmid pLPChyg1 to yield plasmids pLPChd1 and pLPChd2. A restriction site and function map of plasmid pLPChd1 is presented in Figure 13 of the accompanying drawings. The construction of plasmids pLPChd1 and pLPChd2, which differ only with respect to the orientation of the dhfr gene-containing DNA segment, is described in Example 16.

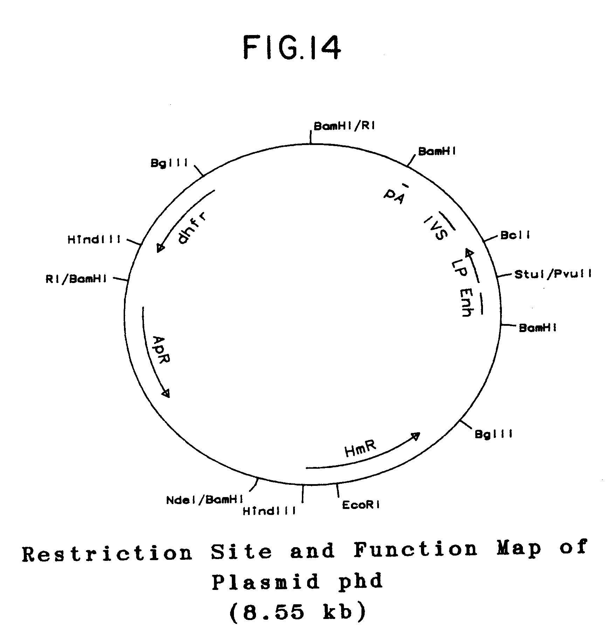

Plasmid pLPChd1 was modified to form plasmid phd, a plasmid that contains both the BK enhancer-adenovirus late promoter cassette and also the hygromycin resistance-conferring and dhfr genes. To construct plasmid phd, plasmid pLPChd1 was prepared from dam- E. coli host cells, digested with restriction enzyme BclI, and recircularized, thus deleting the human protein C-encoding DNA. Plasmid phd contains a single BclI restriction enzyme recognition site, which is conveniently positioned for the insertion of any sequence desired to be expressed from the BK enhancer-adenovirus late promoter of the present invention. A restriction site and function map of plasmid phd is presented in Figure 14 of the accompanying drawings, and the construction protocol for plasmid phd is described in Example 17.

Ultimately, plasmid pShd of the present invention was constructed by ligating the ~2.7 Kb protein S-containing FnuDII-EcoRV restriction fragment from plasmid pHHS-IIa to BclI-digested Klenow-treated plasmid phd. The resulting expression vector utilizes the BK enhancer-adenovirus late promoter cassette to drive expression of human protein S.

An alternative embodiment of the present invention can be constructed as taught above by simply replacing the protein C gene, bounded by BclI restriction sites in plasmid pL133 (which are then treated with Klenow), with the ~2.7 Kb FnuDII-EcoRV restriction fragment isolated from plasmid pHHS-IIa. The resulting expression vector utilizes the SV40 early promoter to drive expression of human protein S. Both of these protein S expression vectors are useful for the production of human protein S.

It is anticipated that protein S can become a valuable adjunct in antithrombotic therapy in several areas. The most obvious is heterozygous protein S deficiency which, as mentioned previously, may involve a sizable number of patients with recurrent deep-vein thromhosis-pulmonary embolism. It is envisioned that protein S be given as intravenous infusions to such patients during acute thrombotic episodes. It is also anticipated that protein S will be useful in the treatment of acquired protein S deficiency so far identified during pregnancy and SLE and in the nephrotic syndrome. It is not unreasonable to anticipate that the list of disease states associated with acquired protein S deficiency may grow to cover many acute and chronic inflammatory conditions as well as autoimmune diseases and cancer. The dose required can be established with certainty only in preclinical and clinical trials and the following calculations should be considered only tentative. The normal level of total protein S in plasma is 30 $\mu$g/ml and the normal level of free protein S is 10-15 $\mu$g/ml. If one desires to elevate circulating levels of protein S in a patient by 5-10 $\mu$g/ml, this would require 30-60 mg administered intravenously provided the volume of distribution of protein S is similar to that of factor IX. The biological half-life of protein S has been established to be 40-48 hours in man. Therefore, infusions every 36-48 hours probably would suffice to cover a patient during the acute phases of his illness.

The possibility exists that protein S might serve a useful function in helping to attach the C4b-binding protein to membrane surfaces at sites where complement activation is occurring. Should this hypothesis prove valid, the therapeutic role of protein S could be expanded to include numerous disease states in which complement activation is an important pathogenetic factor including a wide array of autoimmune and infectious diseases.

The following examples further illustrate the invention disclosed herein. The examples describe the procedures used for the isolation and identification of genetic material encoding human protein S, as well as procedures available for the chemical synthesis of the gene encoding human protein S activity. Procedures used in the construction of expression vectors are also disclosed. Explanations of the procedures are provided where appropriate.

16

Example 1

Screening and Identification of λgt11 Phage Expressing a Protein that Reacts with a Polyclonal Antibody to Human Protein S

The particular λgt11 library used in the present invention is commercially available from Clontech Laboratories, Inc., 922 Industrial Ave., Palo Alto, California 94303. The λgt11 library was generated from human liver cDNA and had a complexity of $5 \times 10^5$ plaques. This library was diluted and about 200,000 phage were plated on each of ten 150 mm dishes containing TY agar (10 g tryptone, 10 g NaCl, 5 g yeast extract and 15 g agar per liter). The procedure used for the screening, described below, was essentially that of the supplier. Immediately after the phage were plated the dishes were incubated at 42°C for 3½ hours. Each plate was overlayed with a nitrocellulose filter saturated with 10 mM IPTG and shifted to 37°C for about 16 hours. The plates were chilled at 4°C for at least 15 minutes before the filters were marked with India ink and transferred to a wash buffer, TBST (50 mM Tris-HCl pH = 7.9, 150 mM NaCl, 0.05% Tween-20). The filters were washed in 200 ml of this buffer three times at five minutes/wash. All washes and reactions were done at room temperature. To reduce non-specific binding of the antibody to the nitrocellulose, the filters were then treated with 20% fetal calf serum in TBST for 30 minutes and washed as before. The initial antibody reaction utilized a polyclonal antibody raised in a goat against human Protein S. These antibodies can be isolated using any of a number of conventional techniques included in Methods in Enzymology 104: 381-387. After one hour of incubation with the primary antibody, the filters were again washed and then treated with a biotinylated secondary antibody (Vector Laboratories, Inc., Burlingame, CA 94010) for 30 minutes. The filters were washed and transferred to TBST containing the avidin-conjugated horseradish peroxidase complex for 30 minutes. For the final wash only TBS is used--the Tween-20 is eliminated. The peroxidase substrate solution was prepared by mixing 3 mg/ml of 4-chloronapthol in methanol with TBS plus 0.01 M imidazole. Three percent hydrogen peroxide was added to 1/250 volume and the solution was immediately added to the filters. The color reaction ("positive" plaques turn purple) was complete within 30 minutes.

Once positive plaques had been identified, the corresponding region of the original plate was removed and added to sterile lambda dilution buffer (10 mM Tris-HCl pH = 7.5, 10 mM MgSO$_4$) and stored at 4°C with a few drops of chloroform. Later these phage were plated at a density of about 5000 plaques per 100 mm dish and screened by the same procedure. Positive plaques were subjected to a third round of screening at a density of about 100 plaques per 100 mm dish and isolated plaques were collected and stored as before.

Example 2

Mapping and Subcloning of the cDNA Region of the Purified Phage

Large amounts of purified phage DNA were isolated using the plate lysate technique (Davis, Botstein and Roth, 1980, Advanced Bacterial Genetics, Cold Spring Harbor Laboratories) as follows. Ten 150 mm dishes were each plated with E. coli infected with about $10^6$ plaque-purified phage. Nearly confluent lysis was achieved after four hours at 42°C. At this point, the plates were chilled for one hour at 4°C, flooded with 10 ml of 0°C lambda dilution buffer, and stored at 4°C overnight. The buffer was carefully removed the next morning and treated with a few drops of chloroform to lyse any remaining cells. Phage particles were removed from the supernatant by centrifugation at 20,000 rpm for 3 hours at 18°C and the pellet was resuspended in 1 ml of lambda dilution buffer. The phage particles were further purified using cesium chloride gradients: the 1 ml phage sample was layered over a step gradient of 1 ml of Solution I (5 M CsCl, 10 mM MgSO$_4$, 0.1 mM Na$_2$EDTA, 10 mM Tris, pH = 8.0); and 3 ml of Solution II (3 M CsCl, 10 mM MgSO$_4$, 0.1 mM Na$_2$EDTA). After 60 minutes at 30,000 rpm (18°C) a faint blue band, corresponding to the phage particles, could be seen at the interphase. Using a syringe and needle, 0.5 ml of the solution was removed at the interphase. At this point, a second gradient could be performed if desired.

To release phage DNA from the protein coat, an equal volume of deionized formamide was mixed with each sample and left at room temperature for more than 30 minutes. The solution was diluted with 0.5 ml of water and the DNA precipitated with two volumes of room temperature ethanol. The DNA was collected by centrifugation for 10 minutes at 10,000 rpm; the pellet was rinsed with 5 ml of 68% ethanol (-20°C), dried and resuspended in 500 μl of TE (10 mM Tris-HCl, pH = 8.0 and 1 mm EDTA). A total of 200-1000 μg of total phage DNA can be recovered.

Phage DNA from each of six positive clones was tested to determine the size(s) of the EcoRI fragments(s) generated from the cDNA insert. About 50 $\mu$g of purified DNA were digested in a volume of 200 $\mu$l containing 20 $\mu$l of 10X EcoRI buffer (100 mM Tris-HCl pH = 7.5, 10 mM MgCl$_2$, 50 mM NaCl) and 50 units of EcoRI for 90 minutes at 37°C. All enzyme units referred to herein, unless otherwise indicated, refer to the unit definitions of New England Biolabs, 32 Tozer Road, Beverly, MA 01915, although the actual source of the enzymes may have been different. The samples were precipitated with ethanol and then separated by electrophoresis on either a 3.5% polyacrylamide gel (29:1, acrylamide:bis-acrylamide) or a 1.0% agarose gel along with appropriate commercially available size markers. The DNA was visualized using ethidium bromide and the size of each band calculated based upon its migration in the gel. Similar digestions were done using XmnI, SstI and KpnI to verify the size of the insert and the orientation of the fragments. One of the largest clones, designated 1-1, contained EcoRI fragments of 1.8 and 0.8 Kb thereby giving a total insert size of 2.6 Kb.

The initial subclones were generated from EcoRI digests of clone 1-1. About 50 $\mu$g of phage DNA were digested with EcoRI as described above for subsequent ligation to about 100 ng of EcoRI-digested, dephosphorylated pBR322.

The ligation was done in a total volume of 20 $\mu$l of 1X ligase buffer (50 mM Tris-HCl, pH = 7.8, 10 mM MgCl$_2$, 20 mM DDT, 1 mM ATP) with 400 units of ligase at 15°C overnight. E. coli RR1 cells, commercially available from Bethesda Research Laboratories, Inc. Gaithersburg MD 20877, were used for transformation according to the protocol provided by Bethesda Research Laboratories and the transformed cells were selected on TY plates containing 50 $\mu$g/ml of ampicillin. The plates were incubated at 37°C overnight. Plasmid DNA was isolated from 12 randomly selected clones and digested with EcoRI to determine the size of the subcloned fragments. The predicted 1.8 and 0.8 Kb fragments were individually subcloned into pBR322 and the resulting plasmids designated pLHS-22 and pLHS-21 respectively. Large amounts of plasmid DNA were purified in preparation for DNA sequencing using chloramphenicol amplification in minimal media followed by alkaline lysis as described by Maniatis, Fritsch, and Sambrook, 1982, Molecular Cloning, Cold Spring Harbor Laboratories.

Example 3

## Isolation of the ~1.8 Kb EcoRI Restriction

## Fragment of Plasmid pLHS-22

Fifty ug in 60 $\mu$l of H$_2$O of the plasmid pLHS-22 were mixed with 20 $\mu$l of 10X EcoRI reaction buffer and 120 $\mu$l of water. This mixture was digested with 50 units of EcoRI at 37°C until the digestion was complete. The EcoRI digested DNA was separated by electrophoresis on a 3.5% polyacrylamide gel until the ~1.8 Kb band was separated from the other digestion products. The gel was then stained in a dilute solution of ethidium bromide so that the bands could be visualized with ultraviolet light.

The region of the gel containing the ~1.8 Kb EcoRI restriction fragment was cut from the gel, placed in a test tube, and broken into small fragments. One ml of extraction buffer (500 mM NH$_4$OAc, 10 mM MgOAc, 1 mM EDTA, and 1% SDS) was added to the tube containing the fragment. The tube was incubated at 37°C overnight. The acrylamide was removed by centrifugation and the supernatant was transferred to a new tube. The acrylamide was washed once with 200 $\mu$l of extraction buffer, centrifuged again, the supernatants combined and passed through a plug of glass wool to remove any remaining contaminants. The DNA was precipitated with two volumes of ethanol, mixed well and kept in a dry ice-ethanol bath for 10 to 30 minutes. The DNA was recovered by centrifugation.

About 15 $\mu$g of the ~1.8 Kb EcoRI restriction fragment were recovered by this procedure. The purified fragment was resuspended in 10 $\mu$l of TE buffer and stored at 4°C.

Example 4

Sequencing Strategy of the cDNA Coding for Human Protein S

The DNA sequence of the cDNA coding for human Protein S was determined in substantial accordance with the teaching of the Maxam and Gilbert protocol (Methods in Enzymology, 1980).

Example 5

Southern Analysis of Protein S Subclones

Plasmid DNA was prepared from several of the protein S EcoRI fragments subcloned into pBR322 (described in Example 3) including the 1.8 and 0.8 Kb fragments from phage 1-1. The DNAs were digested with EcoRI as taught in Example 2 and separated by electrophoresis on a 1.0% agarose gel. The conditions for preparation of the gel, the actual transfer and the hybridization were in substantial accordance with the teaching of Smith and Summers, 1980, Anal Biochem, 109:123-129. Two independent, mixed probes, described in Table 1, were used for the hybridization. As previously discussed, the first probe, #80, was derived from bovine amino acid sequence presented by J. Stenflo, 1985. This probe corresponds to amino acids #83-90 in one of the EGF domains of the bovine molecule. Probe 81, also based upon the bovine sequence, corresponds to the carboxy terminal amino acids #628-634. The 1.8 Kb EcoRI fragment from phage 1-1 hybridized strongly to both of these probes.

Example 6

# Culture of E. coli K12 RR1/pHHS-IIa and Isolation of

# Plasmid pHHS-IIa

## A. Culture of E. coli K12 RR1/pHHS-IIa

Five mls of L-broth (10 g peptone, 10 g NaCl, and 5 g yeast extract) were inoculated with a culture of E. coli RR1/pHHS-IIa (NRRL B-18071) and incubated in an air shaker at 37°C for about 16 hours. A small amount of this culture was streaked on a L-broth agar plate containing 15 μg/ml of tetracycline and grown at 37°C for about 16 hours. An isolated colony was inoculated into 5 ml of L-broth containing 15 μg/ml of tetracycline and grown, with shaking, for about 16 hours at 37°C.

This culture was then used to inoculate one liter of L-broth containing 12 μg/ml tetracycline and incubated in an air-shaker at 37°C until the optical density (O.D.) at 590 nm was ~0.6 absorbance unit, at which time 250 mg of chloramphenicol were added to the culture. The incubation was continued for about 16 hours; the chloramphenicol addition inhibits protein synthesis, and thus inhibits further cell division, but allows plasmid replication to continue.

B. Isolation of Plasmid pHHS-IIa

The culture prepared in Example 6A was centrifuged in a Sorvall GSA rotor (DuPont Co., Instrument Products, Biomedical Division, Newtown, CN 06470) at 6000 rpm for 5 minutes at 4°C. The resulting supernatant was discarded. The cell pellet was frozen at -20°C for a minimum of 2 hours and then thawed. The thawed cell pellet was resuspended in 6.25 ml of a 25% sucrose/50 mM Tris-HCl, pH = 8 solution. After adding and mixing: 1.5 ml of a 10 mg/ml lysozyme solution; 1.25 ml of 0.5 M EDTA, pH = 8.0; the solution was incubated on ice for 10 minutes. Ten ml of lysing solution (0.1% Triton X-100, 0.125 M EDTA, pH = 8, 50 mM Tris, pH = 8.) were added to the lysozyme-treated cells, mixed, and the resulting solution incubated on ice for another 10 minutes.

The cellular debris was removed from the solution by centrifugation at 19,000 rpm for 30 minutes in an SS34 rotor (Sorvall). The solution volume was adjusted to 30 ml with TE and then 28.9 g of CsCl and 3.75 ml of a 10 mg/ml ethidium bromide solution were added and the solution decanted into a Vti50 ultracentrifuge tube (Beckman, Lincolnwood, IL 60646). After sealing the tube, the solution was centrifuged in a Vti50 rotor at 49,000 rpm for ~16 hours. The resulting plasmid band, visualized with ultraviolet light, was isolated. The ethidium bromide was extracted with TE-saturated isobutanol, and the solution diluted with 3 volumes of $H_2O$. One-fortieth volume of 2.0 M NaOAc, pH = 5 and two volumes of ethanol were then added to the solution, followed by incubation overnight at -20°C. The plasmid DNA was pelleted by centrifuging the solution in an SS34 rotor (Sorvall) for 30 minutes at 10,000 rpm. The DNA was resuspended in 5 ml of 0.3 M NaOAc. To this, 10 ml of ethanol were added and the solution was incubated overnight at -20°C. The plasmid DNA was pelleted as taught immediately above.

The ~1 mg of plasmid pHHS-IIa DNA obtained by this procedure was suspended in 1 ml of TE buffer and stored at 4°C. A restriction site and function map of plasmid pHHS-IIa is presented in Figure 2 of the accompanying drawings.

Example 7

Probing Human Liver cDNA Library

As the phage clone 1-1 lacked DNA sequences at both the 5' and 3' ends of the molecule it was necessary to screen again for clones containing additional sequence. For this step a different human liver cDNA library was utilized. This library, which uses the pBR322 vector, is described elsewhere (R.J. Beckmann, et al., Nuc. Acid Res. 13:5233, 1985). Based upon the known DNA sequence, two probes were isolated and designated 21 and 22. The sequence of each is given in Table 2. Probe 21, homologous to a region of the 5' end, consisted of a 110 bp fragment bounded by the recognition sequences for HincII and HindIII. Probe 22 corresponds to a 3' non-translated region of the molecule and was a 130 bp fragment bounded by the recognition sequences for HinfI and EcoRI. These probes were radio-actively labelled and used to probe the cDNA library using standard techniques (Maniatis, et al., 1982). The probes were hybridized and washed at 68°C and all filters were done in duplicate. Several positives were identified by this method. Using restriction enzyme mapping, Southern hybridization and DNA sequencing, one clone, designated pHHS-IIa was identified as containing the complete coding region of the protein S precursor.

Example 8

Preparation of BK Virus DNA

BK virus is obtained from the American Type Culture Collection under the accession number ATCC VR-837. The virus is delivered in freeze-dried form and resuspended in Hank's balanced salts (Gibco, 3175 Staley Road, Grand Island, NY 14072) to a titer of about $10^5$ plaque-forming units (pfu)/ml. The host of choice for the preparation of BK virus DNA is primary human embryonic kidney (PHEK) cells, which can be obtained from Flow Laboratories, Inc., 7655 Old Springhouse Road, McLean, VA 22101, under cataloque number 0-100 or from M.A. Bioproducts under catalogue number 70-151.

About five 75 $mm^2$ polystyrene flasks comprising confluent monolayers of about $10^6$ PHEK cells are used to prepare the virus. About 1 ml of BK virus at a titer of $10^5$ pfu/ml is added to each flask, which is then incubated at 37°C for one hour before fresh culture medium (Delbecco's Modified Eagle's Medium, Gibco, supplemented with 10% fetal bovine serum) is added, and the infected cells are incubated at 37°C for 10-14 days or until the full cytopathogenic effect of the virus is noted. This cytopathogenic effect varies from cell line to cell line and from virus to virus but usually consists of cells rounding up, clumping, and sloughing off the culture disk.

The virus is released from the cells by three freeze-thaw cycles, and the cellular debris is removed by centrifugation at 5000Xg. The virus in 1 liter of supernatant fluid is precipitated and collected by the addition of 100 g of PEG-6000, incubation of the solution for 24 hours at 4°C, and centrifugation at 5000Xg for 20 minutes. The pellet is dissolved in 0.1X SSC buffer (1XSSC = 0.15 M NaCl; 5 mM EDTA; and 50 mM Tris-HCl, pH = 7.8) at 1/100th of the original volume. The virus suspension is layered onto a 15 ml solution of saturated KBr in a tube, which is centrifuged at 75,000Xg for 3 hours. Two bands are evident in the KBr solution after centrifugation. The lower band, which contains the complete virion, is collected and desalted on a Sephadex G-50 column using TE as an elution buffer.

Sodium dodecyl sulfate (SDS) is added to the solution of purified virions obtained from the column to a concentration of 1%; pronase is added to a concentration of 100 $\mu$g/ml, and the solution is incubated at 37°C for 2 hours. Cesium chloride is then added to the solution to a density of 1.56 g/ml, and ethidium bromide is added to the solution to a final concentration of 100 $\mu$g/ml. The solution is centrifuged in a Sorval 865 rotor or similar vertical rotor at 260,000Xg for 24 hours. After centrifugation, the band of virus DNA is isolated and extracted five times with isoamyl alcohol saturated with 100 mM Tris-HCl, pH = 7.8. The solution of BK virus DNA is then dialyzed against TE buffer until the 260 nm/280 nm absorbance ratio of the DNA is between 1.75 and 1.90. The DNA is precipitated by adjusting the NaCl concentration to 0.15 M, adding two volumes of ethanol, incubating the solution at -70°C for at least 2 hours, and centrifuging the solution at 12,000Xg for 10 minutes. The resulting pellet of BK virus DNA is suspended in TE buffer at a concentration of 1 mg/ml.

Example 9

Construction of Plasmids pBKE1 and pBKE2

About one μg of the BK virus DNA prepared in Example 8 in one μl of TE buffer was dissolved in 2 μl of 10X EcoRI buffer (1.0 M Tris-HCl, pH = 7.5; 0.5 M NaCl; 50 mM MgCl₂; and 1 mg/ml BSA) and 15 μl of H₂O. About 2 μl (~10 units) of restriction enzyme EcoRI were added to the solution of DNA, and the resulting reaction was incubated at 37°C for two hours.

About 1 μg of plasmid pUC8 (available from Pharmacia P-L Biochemicals, 800 Centennial Ave., Piscataway, NJ 08854) in 1 μl of TE buffer was digested with EcoRI in substantial accordance with the procedure used to prepare the EcoRI-digested BK virus DNA. The EcoRI-digested plasmid pUC8 DNA was diluted to 100 μl in TE buffer; ~0.06 units of calf-intestinal alkaline phosphatase were added to the solution, and the resulting reaction was incubated at 37°C for 30 minutes. The solution was adjusted to contain 1X SET (5 mM Tris-HCl, pH = 7.8; 5 mM EDTA; and 150 mM NaCl), 0.3M NaOAc, and 0.5% SDS and then incubated at 65°C for 45 minutes. The phosphatase treatment prevents the pUC8 DNA from self ligating.

The EcoRI-digested BK virus and plasmid pUC8 DNA were extracted first with buffered phenol and then with chloroform. The DNA was collected by adjusting the NaCl concentration of each DNA solution to 0.25 M, adding two volumes of ethanol, incubating the resulting mixtures in a dry ice-ethanol bath for 5 minutes, and centrifuging to pellet the DNA. The supernatants were discarded, and the DNA pellets were rinsed with 70% ethanol, dried, and resuspended in 10 μl and 30 μl of TE buffer for the BK and plasmid pUC8 samples, respectively.

About 3 μl of H₂O and 1 μl of 10X ligase buffer were added to a mixture of 2 μl of the EcoRI-digested BK virus and 1 μl of the EcoRI-digested plasmid pUC8 DNA. One μl (~1000 units) of T4 DNA ligase were added to the solution of DNA, and the resulting reaction was incubated at 16°C overnight. The ligated DNA constituted the desired plasmids pBKE1 and pBKE2, which differ only with respect to the orientation of the inserted BK virus DNA. A restriction site and function map of plasmid pBKE1 is presented in Figure 3 of the accompanying drawings.

A 50 ml culture of E. coli K12 JM103, available from Pharmacia P-L Biochemicals, in L-broth was grown to an optical density at 650 nanometers (O.D.₆₅₀) of approximately 0.4 absorbance units. The culture was chilled on ice for ten minutes, and the cells were collected by centrifugation. The cell pellet was resuspended in 25 ml of cold 100 mM MgCl₂ and incubated on ice for 25 minutes. The cells were once again pelleted by centrifugation, and the pellet was resuspended in 2.5 ml of cold 100 mM CaCl₂ and incubated for 30 minutes on ice. After the incubation, the cells are competent for the uptake of transforming DNA. This procedure is used in the following examples requiring the use of competent cells.

Two hundred μl of this cell suspension were mixed with the ligated DNA prepared above and incubated on ice for 30 minutes. At the end of this period, the cells were placed in a water bath at 42°C for 2 minutes and then returned to the ice for an additional 10 minutes. The cells were collected by centrifugation and resuspended in one ml of L broth and incubated at 37°C for 1 hour.

Aliquots of the cell mixture were plated on L-agar (L broth with 15 grams of agar per liter) plates containing 100 μg ampicillin/ml, 40 μg X-gal/ml, and 40 μg IPTG/ml. The plates were incubated at 37°C overnight. Colonies that contain a plasmid without an insert, such as E. coli K12 JM103/pUC8, appear blue on these plates. Colonies that contain a plasmid with an insert, such as E. coli K12 JM103/pBKE1, are white. Several white colonies were selected and screened by restriction enzyme analysis of their plasmid DNA for the presence of the ~5.2 Kb EcoRI restriction fragment of BK virus. Plasmid DNA was obtained from the E. coli K12 JM103/pBKE1 and E. coli K12 JM103/pBKE2 cells in substantial accordance with the procedure for isolating plasmid DNA that is described in the following example, although the procedure is done on a smaller scale, and the CsCl gradient steps are omitted, when the plasmid. DNA is isolated only for restriction enzyme analysis.

Example 10

Construction of Plasmids pBKneo1 and pBKneo2

E. coli K12 HB101/pdBPV-MMTneo cells are obtained in lyophil form from the American Type Culture Collection under the accession number ATCC 37224. The lyophilized cells are plated on L-agar plates containing 100 μg/ml ampicillin and incubated at 37°C to obtain single colony isolates.

One liter of L broth containing 50 μg/ml ampicillin was inoculated with a colony of E. coli K12 HB101/pdBPV-MMTneo and incubated in an air-shaker at 37°C until the O.D.₅₉₀ was ~1 absorbance unit, at which time 150 mg of chloramphenicol were added to the culture. The incubation was continued for about 16 hours; the chloramphenicol addition inhibits protein synthesis, and thus inhibits further cell division, but allows plasmid replication to continue.

The culture was centrifuged in a Sorvall GSA rotor (DuPont Co., Instrument Products, Biomedical Division, Newtown, CN 06470) at 6000 rpm for 5 minutes at 4°C. The resulting supernatant was discarded, and the cell pellet was washed in 40 ml of TES buffer (10 mM Tris-HCl, pH=7.5; 10 mM NaCl; and 1 mM EDTA) and then repelleted. The supernatant was discarded, and the cell pellet was frozen in a dry ice-ethanol bath and then thawed. The thawed cell pellet was resuspended in 10 ml of a solution of 25% sucrose and 50 mM EDTA. About 1 ml of a 5 mg/ml lysozyme solution; 3 ml of 0.25 M EDTA, pH=8.0; and 100 $\mu$l of 10 mg/ml RNAse A were added to the solution, which was then incubated on ice for 15 minutes. Three ml of lysing solution (prepared by mixing 3 ml 10% Triton-X 100; 75 ml 0.25 M EDTA, pH=8.0; 15 ml of 1 M Tris-HCl, pH=8.0; and 7 ml of water) were added to the lysozyme-treated cells, mixed, and the resulting solution incubated on ice for another 15 minutes. The lysed cells were frozen in a dry ice-ethanol bath and then thawed.

The cellular debris was removed from the solution by centrifugation at 25,000 rpm for 40 minutes in an SW27 rotor (Beckman, 7360 N. Lincoln Ave., Lincolnwood, IL 60646) and by extraction with buffered phenol. About 30.44 g of CsCl and ~1 ml of a 5 mg/ml ethidium bromide solution were added to the cell extract and the volume of the solution was adjusted to 40 ml with TES buffer. The solution was decanted into a VTi50 ultra-centrifuge tube (Beckman), which was then sealed and centrifuged in a VTi50 rotor at 42,000 rpm for ~16 hours. The resulting plasmid band, visualized with ultraviolet light, was isolated and then placed in a Ti75 tube and rotor (Beckman) and centrifuged at 50,000 rpm for 16 hours. Any necessary volume adjustments were made using TES containing 0.761 g/ml CSCl: The plasmid band was again isolated, extracted with salt-saturated isopropanol to remove the ethidium bromide, and diluted 1:3 with TES buffer. Two volumes of ethanol were then added to the solution, which was then incubated overnight at -20°C. The plasmid DNA was pelleted by centrifuging the solution in an SS34 rotor (Sorvall) for 15 minutes at 10,000 rpm.

The ~1 mg of plasmid pdBPV-MMTneo DNA obtained by this procedure was suspended in 1 ml of TE buffer and stored at -20°C. The foregoing plasmid isolation procedure is generally used throughout the following examples when large amounts of very pure plasmid DNA are desired. The procedure can be modified to rapidly obtain a smaller, less pure amount of DNA, such as is needed when screening transformants for the presence of a given plasmid, by using only about 5 ml of cultured cells, lysing the cells in an appropriately scaled-down amount of lysis buffer, and replacing the centrifugation steps with phenol and chloroform extractions.

About 5 $\mu$g (5 $\mu$l) of the plasmid pdBPV-MMTneo DNA prepared above and five $\mu$g (5 $\mu$l) of the BK virus DNA prepared in Example 8 were each digested at 37°C for 2 hours in a solution containing 2 $\mu$l of 10X BamHI buffer (1.5 M NaCl; 60 mM Tris-HCl, pH=7.9; 60 mM MgCl$_2$; and 1 mg/ml BSA), 1 $\mu$l of restriction enzyme BamHI, and 7 $\mu$l of H$_2$O. The reaction was stopped by an extraction with an equal volume of phenol, followed by two extractions with chloroform. Each BamHI-digested DNA was then precipitated, collected by centrifugation, and resuspended in 5 $\mu$l of H$_2$O.

About 1 $\mu$l of 10X ligase buffer was added to a mixture of BamHI-digested plasmid pdBPV-MMTneo (1 $\mu$l) and BamHI-digested BK virus DNA (1 $\mu$l). After 1 $\mu$l (~1000 units) of T4 DNA ligase and 6 $\mu$l of H$_2$O were added to the mixture of DNA, the resulting reaction was incubated at 16°C overnight. The ligated DNA constituted the desired plasmids pBKneo1 and pBKneo2, which differ only with respect to the orientation of the BK virus DNA. A restriction site and function map of plasmid pBKneo1 is presented in Figure 4 of the accompanying drawings.

E. coli K12 HB101 cells are available in lyophilized form from the NRRL under the accession number NRRL 5-15626. E. coli K12 HB101 cells were cultured, made competent for transformation, and transformed with the ligated DNA prepared above. The transformed cells were plated on L-agar plates containing 100 $\mu$g/ml ampicillin. E. coli K12 HB101/psKneo1 and E. coli K12/pBKneo2 transformants were identified by their ampicillin-resistant phenotype and by restriction enzyme analysis of their plasmid DNA.

Example 11

Construction of Plasmid pBLcat

A. Construction of Intermediate Plasmid pLPcat

The virion DNA of adenovirus 2 (Ad2) is a double-stranded linear molecule about 35.94 Kb in size. The Ad2 late promoter can be isolated on an ~0.316 Kb AccI-PvuII restriction fragment of the Ad2 genome; this ~0.32 Kb restriction fragment corresponds to the sequence between nucleotide positions 5755 and 6071 of the Ad2 genome. To isolate the desired ~0.32 Kb AccI-PvuII restriction fragment, Ad2 DNA is first digested with restriction enzyme BalI, and the ~2.4 Kb BalI restriction fragment that comprises the entire sequence of the ~0.32 Kb AccI-PvuII restriction fragment is isolated. Then, the ~2.4 Kb BalI restriction fragment is digested with AccI and PvuII to obtain the desired fragment.

About 50 $\mu$g of Ad2 DNA (available from BRL) are dissolved in 80 $\mu$l of $H_2O$ and 10 $\mu$l of 10X BalI buffer (100 mM Tris-HCl, pH = 7.6; 120 mM $MgCl_2$; 100 mM DTT; and 1 mg/ml BSA). About 10 $\mu$l (~20 units) of restriction enzyme BalI are added to the solution of Ad2 DNA, and the resulting reaction is incubated at 37°C for 4 hours.

The BalI-digested DNA is loaded onto an agarose gel and electrophoresed until the restriction fragments are well separated. Visualization of the electrophoresed DNA is accomplished by staining the gel in a dilute solution (0.5 $\mu$g/ml) of ethidium bromide and exposing the stained gel to long-wave ultraviolet (UV) light. One method to isolate DNA from agarose is as follows. A small slit is made in the gel in front of the desired fragment, and a small piece of NA-45 DEAE membrane (Schleicher and Schuell, Keene, NH 03431) is placed in each slit. Upon further electrophoresis, the DNA non-covalently binds to the DEAE membrane. After the desired fragment is bound to the DEAE membrane, the membrane is removed and rinsed with low-salt buffer (100 mM KCl; 0.1 mM EDTA; and 20 mM Tris-HCl, pH = 8). Next, the membrane is placed in a small tube and immersed in high-salt buffer (1 M NaCl; 0.1 mM EDTA; and 20 mM Tris-HCl, pH = 8) and then incubated at 65°C for one hour to remove the DNA from the DEAE paper. After the 65°C incubation, the incubation buffer is collected and the membrane rinsed with high-salt buffer. The high-salt rinse solution is pooled with the high-salt incubation buffer.

The volume of the high salt-DNA solution is adjusted so that the NaCl concentration is 0.25 M, and then three volumes of cold, absolute ethanol are added to the solution. The resulting solution is mixed and placed at -70°C for 10-20 minutes. The solution is then centrifuged at 15,000 rpm for 15 minutes. After another precipitation to remove residual salt, the DNA pellet is rinsed with ethanol, dried, resuspended in 20 $\mu$l of TE buffer, and constitutes about 3 $\mu$g of the desired restriction fragment of Ad2. The purified fragment obtained is dissolved in 10 $\mu$l of TE buffer.

About 6 $\mu$l of $H_2O$ and 2 $\mu$l of 10X AccI buffer (60 mM NaCl; 60 mM Tris-HCl, pH = 7.5; 60 mM $MgCl_2$; 60 mM DTT; and 1 mg/ml BSA) are added to the solution of the ~2.4 Kb BalI restriction fragment of Ad2. After the addition of about 2 $\mu$l (~10 units) of restriction enzyme AccI to the solution of DNA, the reaction is incubated at 37°C for 2 hours. After the AccI digestion, the DNA is collected by ethanol precipitation and resuspended in 16 $\mu$l of $H_2O$ and 2 $\mu$l of 10X PvuII buffer (600 mM NaCl; 60 mM Tris-HCl, pH = 7.5; 60 mM $MgCl_2$; 60 mM DTT; and 1 mg/ml BSA). After the addition of about 2 $\mu$l (about 10 units) of restriction enzyme PvuII to the solution of DNA, the reaction is incubated at 37°C for 2 hours.

The AccI-PvuII-digested, ~2.4 Kb BalI restriction fragment of Ad2 is loaded onto an ~6% polyacrylamide gel and electrophoresed until the ~0.32 Kb AccI-PvuII restriction fragment that comprises the Ad2 late promoter is separated from the other digestion products. The gel is stained with ethidium bromide and viewed using UV light, and the segment of gel containing the ~0.32 Kb AccI-PvuII restriction fragment is cut from the gel, crushed, and soaked overnight at room temperature in ~250 $\mu$l of extraction buffer. The following morning, the mixture is centrifuged, and the pellet is discarded. The DNA in the supernatant is precipitated with ethanol; about 2 $\mu$g of tRNA are added to ensure complete precipitation of the desired fragment. About 0.2 $\mu$g of the ~0.32 Kb AccI-PvuII restriction fragment are obtained and suspended in 7 $\mu$l of $H_2O$.

About 0.25 $\mu$g (in 0.5 $\mu$l) of BclI linkers (5'-CTGATCAG-3', available from New England Biolabs), were kinased and prepared for ligation by the following procedure. Four $\mu$l of linkers (~2 $\mu$g) were dissolved in 20.15 $\mu$l of $H_2O$ and 5 $\mu$l of 10X kinase buffer (500 mM Tris-HCl, pH = 7.6 and 100 mM $MgCl_2$), incubated at 90°C for two minutes, and then cooled to room temperature. Five $\mu$l of [$\gamma$-$^{32}$P]-ATP (~20 $\mu$Ci), 2.5 $\mu$l of 1 M DTT, and 5 $\mu$l of polynucleotide kinase (~10 units) were added to the mixture, which was then incubated at 37°C for 30 minutes. Then, 3.35 $\mu$l of 0.01 M ATP and 5 $\mu$l of kinase were added, and the reaction was continued for another 30 minutes at 37°C. The radioactive ATP aids in determining whether the linkers have ligated to the target DNA.

The kinased linkers were added to the solution of the ~0.32 kb AccI-PvuII restriction fragment before 1 µl (~1000 units) of T4 DNA ligase and 1 µl of 10X ligase buffer were added to the solution of DNA. The resulting reaction was incubated at 16°C overnight. The BclI linkers could only ligate to the PvuII end of the AccI-PvuII restriction fragment. DNA sequencing later revealed that four BclI linkers attached to the PvuII end of the AccI-PvuII restriction fragment. These extra BclI linkers can be removed by BclI digestion and religation; however, the extra BclI linkers were not removed as the linkers do not interfere with the proper functioning of the vectors.

E. coli K12 HB101/pSV2cat cells are obtained in lyophilized form from the ATCC under the accession number ATCC 37155, and plasmid pSV2cat DNA was isolated from the cells. A restriction site and function map of plasmid pSV2cat is presented in Figure 5 of the accompanying drawings. About 1 mg of plasmid pSV2cat DNA is obtained and dissolved in 1 ml of TE buffer. About 3 µg (3 µl) of the plasmid pSV2cat DNA were added to 2 µl of 10X AccI buffer and 16 µl of H₂O, and then, 3 µl (about 9 units) of restriction enzyme AccI were added to the solution of pSV2cat DNA, and the resulting reaction was incubated at 37°C for 2 hours. The AccI-digested plasmid pSV2cat DNA was then digested with restriction enzyme StuI by adding 3 µl of 10X StuI buffer (1.0 M NaCl; 100 mM Tris-HCl, pH = 8.0; 100 mM MgCl₂; 60 mM DTT; and 1 mg/ml BSA), 5 µl of H₂O, and about 2 µl (about 10 units) of restriction enzyme StuI. The resulting reaction was incubated at 37°C for 2 hours. The reaction was terminated by extracting the reaction mixture once with phenol, then twice with chloroform. About 0.5 µg of the desired fragment was obtained and dissolved in 20 µl of TE buffer.

About 4 µl of the AccI-StuI-digested plasmid pSV2cat DNA were mixed with about 7 µl of the ~0.32 Kb AccI-PvuII (with BclI linkers attached) restriction fragment of Ad2, and after the addition of 3 µl of 10X ligase buffer, 15 µl of H₂O, and 2 µl (about 1000 units) of T4 DNA ligase, the ligation reaction was incubated at 16°C overnight. The ligated DNA constituted the desired plasmid pLPcat, a plasmid that comprises the Ad2 late promoter positioned so as to drive transcription, and thus expression, of the chloramphenicol acetyltransferase gene. A restriction site and function map of plasmid pLPcat is presented in Figure 6 of the accompanying drawings.

The ligated DNA was used to transform E. coli K12 HB101 cells and the transformed cells were plated on L agar containing 50 µg/ml ampicillin; restriction enzyme analysis of plasmid DNA was used to identify the E. coli K12 HB101/pLPcat transformants. Plasmid pLPcat DNA was then isolated from the transformants for use in subsequent vector constructions.

B. Final Construction of Plasmid pBLcat

About 88 µg of plasmid pBKneo1 DNA in 50 µl of TE buffer were added to 7.5 µl of 10X AccI buffer, 30 µl of H₂O, and 15 µl (about 75 units) of restriction enzyme AccI, and the resulting reaction was incubated at 37°C for 2 hours. The AccI-digested BK virus DNA was loaded on an agarose gel, and the ~1.4 Kb fragment that contains the BK enhancer was separated from the other digestion products. The ~1.4 Kb AccI restriction fragment was then isolated in substantial accordance with the procedure described in Example 9. About 5 µg of the fragment were resuspended in 5 µl of 10X PvuII buffer, 45 µl of H₂O, and 5 µl (about 25 units) of restriction enzyme PvuII, and the resulting reaction was incubated at 37°C for 2 hours. The PvuII-digested DNA was then isolated and prepared for ligation in substantial accordance with the procedure of Example 9. About 2 µg of the desired ~1.28 Kb AccI-PvuII fragment were obtained and dissolved in 5 µl of TE buffer.

About 1 µg of plasmid pLPcat DNA was dissolved in 5 µl of 10X AccI buffer and 40 µl of H₂O. About 5 µl (~25 units) of restriction enzyme AccI were added to the solution of plasmid pLPcat DNA, and the resulting reaction was incubated at 37°C. The AccI-digested plasmid pLPcat DNA was precipitated with ethanol and resuspended in 5 µl of 10X StuI buffer, 40 µl of H₂O, and 5 µl (about 25 units) of restriction enzyme StuI, and the resulting reaction was incubated at 37°C for 2 hours. The AccI-StuI-digested plasmid pLPcat DNA was precipitated with ethanol several times to purify the ~4.81 Kb AccI-StuI restriction fragment that comprises the E. coli origin of replication and Ad2 late promoter away from the other digestion product, a restriction fragment about 16 bp in size. About 1 µg of the desired ~4.81 Kb restriction fragment was obtained and dissolved in 20 µl of TE buffer.

The 5 µl of ~4.81 Kb AccI-StuI restriction fragment of plasmid pLPcat were added to 5 µl of ~1.28 Kb AccI-PvuII restriction fragment of BK virus. After the addition of 3 µl of 10X ligase buffer, 15 µl of H₂O, and 2 µl (about 1000 units) of T4 DNA ligase to the mixture of DNA, the resulting ligation reaction was incubated at 16°C overnight. The ligated DNA constituted the desired plasmid pBLcat. A restriction site and function map of plasmid pBLcat is presented in Figure 7 of the accompanying drawings.

The ligated DNA was used to transform E. coli K12 HB101 cells and E. coli K12 HB101/pBLcat transformants were identified by restriction enzyme analysis of their plasmid DNA. Next, plasmid pBLcat DNA was prepared for use in subsequent vector constructions.

Example 12

Construction of Plasmid pL133

A. Construction of Intermediate Plasmid pSV2-HPC8

Plasmid pHC7 comprises a DNA sequence that encodes human protein C. One liter of L-broth containing 15 $\mu$g/ml tetracycline was inoculated with a culture of E. coli K12 RR1/pHC7 (NRRL B-15926), and plasmid pHC7 DNA was isolated and purified in substantial accordance with the procedure of Example 10. About 1 mg of plasmid pHC7 DNA was obtained by this procedure, suspended in 1 ml of TE buffer, and stored at -20°C. A restriction site and function map of plasmid pHC7 is presented in Figure 8 of the accompanying drawings.

Fifty $\mu$l of the plasmid pHC7 DNA were mixed with 5 $\mu$l (~50 units) of restriction enzyme BanI, 10 $\mu$l of 10X BanI reaction buffer (1.5 M NaCl; 60 mM Tris-HCl, pH = 7.9; 60 mM $MgCl_2$; and 1 mg/ml BSA), and 35 $\mu$l of $H_2O$ and incubated until the digestion was complete. The BanI-digested plasmid pHC7 DNA was then electrophoresed on a 3.5% polyacrylamide gel, until the ~1.25 Kb BanI restriction fragment was separated from the other digestion products. The region of the gel containing the ~1.25 Kb BanI restriction fragment was isolated according to the teaching of Example 3.

Approximately 8 $\mu$g of the ~1.25 Kb BanI restriction fragment were obtained by this procedure. The purified fragment was suspended in 10 $\mu$l of TE buffer and stored at -20°C. The BanI restriction fragment had to be modified by the addition of a linker to construct plasmid pSV2-HPC8. The DNA fragments used in the construction of the linker were synthesized either by using a Systec 1450A DNA Synthesizer or an ABS 380A DNA Synthesizer.

Five hundred picomoles of each single strand of the linker were kinased in 20 $\mu$l of reaction buffer, which contained 15 units (~0.5 $\mu$l) T4 polynucleotide kinase, 2 $\mu$l 10X ligase buffer, 10 $\mu$l of 500 $\mu$M ATP, and 7.5 $\mu$l of $H_2O$. The kinase reaction was incubated at 37°C for 30 minutes, and the reaction was terminated by incubation at 100°C for 10 minutes. In order to ensure complete kination, the reaction was chilled on ice, 2 $\mu$l of 0.2 M dithiothreitol, 2.5 $\mu$l of 5 mM ATP, and 15 units of T4 polynucleotide kinase were added to the reaction mixture and mixed, and the reaction mixture was incubated another 30 minutes at 37°C. The reaction was stopped by another 10 minute incubation at 100°C and then chilled on ice.

Although kinased separately, the two single strands of the DNA linker were mixed together after the kinase reaction. To anneal the strands, the kinase reaction mixture was incubated at 100°C for 10 minutes in a water bath containing ~150 ml of water. After this incubation, the water bath was shut off and allowed to cool to room temperature, a process taking about 3 hours. The water bath, still containing the tube of kinased DNA, was then incubated at 4°C overnight. This process annealed the single strands. The linker constructed had the following structure:

```
5'-AGCTTTGATCAG-3'
   |||||||
 3'-AACTAGTCCACG-5'
```

The linker was stored at -20°C until use.

The ~8 $\mu$g of ~1.25 Kb BanI fragment were added to and mixed with the ~50 $\mu$l of linker (~500 picomoles), 1 $\mu$l of T4 DNA ligase (~500 units), 10 $\mu$l of 10X ligase buffer, and 29 $\mu$l of $H_2O$, and the resulting ligation reaction was incubated at 4°C overnight. The ligation reaction was stopped by a 10 minute incubation at 65°C. The DNA was pelleted by adding NaOAc to a final concentration of 0.3 M, adding 2 volumes of ethanol, chilling in a dry ice-ethanol bath, and then centrifuging the solution.

The DNA pellet was dissolved in 10 $\mu$l of 10X ApaI reaction buffer (60 mM NaCl; 60 mM Tris-HCl, pH = 7.4; 60 mM $MgCl_2$; and 60 mM 2-mercaptoethanol), 5 $\mu$l (~50 units) of restriction enzyme ApaI, and 85 $\mu$l of $H_2O$, and the reaction was placed at 37°C for two hours. The reaction was then stopped and the DNA pelleted as above. The DNA pellet was dissolved in 10 $\mu$l of 10X HindIII reaction buffer, 5 $\mu$l (~50 units) of restriction enzyme HindIII, and 85 $\mu$l of $H_2O$, and the reaction was placed at 37°C for two hours. After the HindIII digestion, the reaction mixture was loaded onto a 3.5% polyacrylamide gel, and the desired ~1.23 Kb HindIII-ApaI restriction fragment was isolated. Approximately 5 $\mu$g of the desired fragment were obtained, suspended in 10 $\mu$l of TE buffer, and stored at -20°C.

Fifty $\mu$l of plasmid pHC7 DNA were mixed with 5 $\mu$l (~50 units) of restriction enzyme PstI, 10 $\mu$l of 10X PstI reaction buffer (1.0 M NaCl; 100 mM Tris-HCl, pH = 7.5; 100 mM MgCl$_2$; and 1 mg/ml BSA), and 35 $\mu$l of H$_2$O and incubated at 37°C for two hours. The PstI-digested plasmid pHC7 DNA was then electrophoresed on a 3.5% polyacrylamide gel, and the desired ~0.88 Kb fragment was purified in substantial accordance with the procedure described above. Approximately 5 $\mu$g of the desired fragment were obtained, suspended in 10 $\mu$l of TE buffer, and stored at -20°C.

The ~5 $\mu$g of ~0.88 Kb PstI fragment were added to and mixed with ~50 $\mu$l of the following linker, which was constructed on an automated DNA synthesizer:

```
5'-GTGATCAA-3'
  ||||||||
3'-ACGTCACTAGTTCTAG-5'
```

About 1 $\mu$l of T4 DNA ligase (~10 units), 10 $\mu$l 10X ligase buffer, and 29 $\mu$l H$_2$O were added to the mixture of DNA, and the resulting ligation reaction was incubated at 4°C overnight.

The ligation reaction was stopped by a 10 minute incubation at 65°C. After precipitation of the ligated DNA, the DNA pellet was dissolved in 10 $\mu$l of 10X ApaI reaction buffer, 5 $\mu$l (~50 units) of restriction enzyme ApaI, and 85 $\mu$l of H$_2$O, and the reaction was placed at 37° for two hours. The reaction was then stopped and the DNA pelleted once again. The DNA pellet was dissolved in 10 $\mu$l 10X BglII reaction buffer (1 M NaCl; 100 mM Tris-HCl, pH = 7.4; 100 mM MgCl$_2$; 100 mM 2-mercaptoethanol; and 1 mg/ml BSA), 5 $\mu$l (~50 units) of restriction enzyme BglII, and 85 $\mu$l H$_2$O, and the reaction was placed at 37°C for two hours. After the BglII digestion, the reaction mixture was loaded onto a 3.5% polyacrylamide gel to isolate the desired ~0.19 Kb ApaI-BglII restriction fragment. Approximately 1 $\mu$g of the desired fragment was obtained, suspended in 10 $\mu$l of TE buffer, and stored at -20°C.

Approximately 10 $\mu$g of plasmid pSV2gpt DNA (ATCC 37145) were dissolved in 10 $\mu$l of 10X HindIII reaction buffer, 5 $\mu$l (~50 units) of restriction enzyme HindIII, and 85 $\mu$l of H$_2$O, and the reaction was placed at 37°C for 2 hours. The reaction mixture was then made 0.25 M in NaOAc, and after the addition of two volumes of ethanol and incubation in a dry ice-ethanol bath, the DNA was pelleted by centrifugation. The DNA pellet was dissolved in 10 $\mu$l of 10X BglII buffer, 5 $\mu$l (~50 units) of restriction enzyme BglII, and 85 $\mu$l of H$_2$O, and the reaction was placed at 37°C for two hours. After the BglII digestion, the reaction mixture was loaded onto a 1% agarose gel, and the fragments were separated by electrophoresis. The gel was stained with ethidium bromide and viewed under ultraviolet light, and the band containing the desired ~5.1 Kb HindIII-BglII fragment was cut from the gel and placed in dialysis tubing, and electrophoresis was continued until the DNA was out of the agarose. The buffer containing the DNA from the dialysis tubing was extracted with phenol and CHCl$_3$, and then, the DNA was precipitated. The pellet was resuspended in 10 $\mu$l of TE buffer and constituted ~5 $\mu$g of the desired ~5.1 Kb HindIII-BglII restriction fragment of plasmid pSV2gpt.

Two $\mu$l of the ~1.23 Kb HindIII-ApaI restriction fragment, 3 $\mu$l of the ~0.19 Kb ApaI-BglII fragment, and 2 $\mu$l of the ~5.1 Kb HindIII-BglII fragment were mixed together and then incubated with 10 $\mu$l of 10X ligase buffer, 1 $\mu$l of T4 DNA ligase (~500 units), and 82 $\mu$l of H$_2$O at 16°C overnight. The ligated DNA constituted the desired plasmid pSV2-HPC8; a restriction site and function map of the plasmid is presented in Figure 8 of the accompanying drawings.

E. coli K12 RR1 (NRRL B-15210) cells were made competent for transformation and the ligated DNA prepared above was used to transform the cells. Aliquots of the transformation mix were plated on L-agar plates containing 100 $\mu$g/ml ampicillin. The plates were then incubated at 37°C. E. coli K12 RR1/pSV2-HPC8 transformants were verified by restriction enzyme analysis of their plasmid DNA.

B. Final Construction of Plasmid pL133

Fifty $\mu$g of plasmid pSV2-HPC8 were dissolved in 10 $\mu$l of 10X HindIII reaction buffer, 5 $\mu$l (~50 units) of restriction enzyme HindIII, and 85 $\mu$l of H$_2$O, and the reaction was incubated at 37°C for two hours. After the HindIII digestion, the DNA was precipitated, and the DNA pellet was dissolved in 10 $\mu$l 10X SalI reaction buffer (1.5 M NaCl; 60 mM Tris-HCl, pH = 7.9; 60 mM MgCl$_2$; 60 mM 2-mercaptoethanol; and 1 mg/ml

BSA), 5 $\mu$l (~50 units) of restriction enzyme SalI, and 85 $\mu$l of H$_2$O. The resulting SalI reaction mixture was incubated for 2 hours at 37°C. The HindIII-SalI-digested plasmid pSV2-HPC8 was loaded onto a 3.5% polyacrylamide gel and electrophoresed until the desired ~0.29 Kb HindIII-SalI restriction fragment was separated from the other reaction products. The desired fragment was isolated from the gel; about 2 $\mu$g of the fragment were obtained and suspended in 10 $\mu$l of TE buffer.

Fifty $\mu$g of plasmid pSV2-HPC8 were dissolved in 10 $\mu$l of 10X BglII reaction buffer, 5 $\mu$l (50 units) of restriction enzyme BglII, and 85 $\mu$l of H$_2$O, and the reaction was incubated at 37°C for two hours. After the BglII digestion, the DNA was precipitated, and the DNA pellet was dissolved in 10 $\mu$l of 10X SalI reaction buffer, 5 $\mu$l (~50 units) of restriction enzyme SalI, and 85 $\mu$l of H$_2$O. The resulting SalI reaction mixture was incubated for 2 hours at 37°C. The SalI-BglII-digested plasmid pSV2-HPC8 was loaded onto a 3.5% polyacrylamide gel and electrophoresed until the desired ~1.15 Kb SalI-BglII restriction fragment was separated from the other reaction products. The ~1.15 Kb SalI-BglII restriction fragment was isolated from the gel; about 8 $\mu$g of fragment were obtained and suspended in 10 $\mu$l of TE buffer.

Approximately 10 $\mu$g of plasmid pSV2-$\beta$-globin DNA (NRRL B-15928) were dissolved in 10 $\mu$l of 10X HindIII reaction buffer, 5 $\mu$l (~50 units) of restriction enzyme HindIII, and 85 $\mu$l of H$_2$O, and the reaction was placed at 37°C for 2 hours. The reaction mixture was then made 0.25 M in NaOAc, and after the addition of two volumes of ethanol and incubation in a dry ice-ethanol bath, the DNA was pelleted by centrifugation. The HindIII-digested plasmid pSV2-$\beta$-globin was dissolved in 10 $\mu$l of 10X BglII buffer, 5 $\mu$l (~50 units) of restriction enzyme BglII, and 85 $\mu$l of H$_2$O, and the reaction was placed at 37°C for two hours. After the BglII digestion, the reaction mixture was loaded onto a 1% agarose gel, and the fragments were separated by electrophoresis. The desired ~4.2 Kb HindIII-BglII restriction fragment was isolated from the gel; about 5 $\mu$g of the desired fragment were obtained and suspended in 10 $\mu$g of TE buffer.

Two $\mu$g of the ~0.29 Kb HindIII-SalI fragment of plasmid pSV2-HPC8, 2 $\mu$l of the ~1.15 Kb SalI-BglII fragment of plasmid pSV2-HPC8, and 2 $\mu$l of the ~4.2 Kb HindIII-BglII fragment of plasmid pSV2-$\beta$-globin were mixed together and ligated in substantial accordance with the procedure of Example 12A. The ligated DNA constituted the desired plasmid pL133; Figure 8 illustrates by flow chart the construction of plasmid pL133 from the various starting materials described throughout this example. The desired E. coli K12 RR1/pL133 transformants were constructed and plasmid DNA was isolated for use in the construction of plasmid pLPC.

Example 13

Construction of Plasmid pLPC

About 20 $\mu$g of plasmid pBLcat DNA were dissolved in 10 $\mu$l of 10X HindIII buffer and 80 $\mu$l of H$_2$O. About 10 $\mu$l (~100 units) of restriction enzyme HindIII were added to the solution of plasmid pBLcat DNA, and the resulting reaction was incubated at 37°C for 2 hours. The HindIII-digested plasmid pBLcat DNA was loaded onto an agarose gel and electrophoresed until the ~0.87 Kb HindIII restriction fragment that comprises the BK enhancer and Ad2 late promoter was separated from the other digestion products; then, the ~0.87 Kb fragment was isolated and prepared for ligation in substantial accordance with the procedure of Example 9. About 2 $\mu$g of the desired fragment were obtained and dissolved in 5 $\mu$l of TE buffer.

About 1.5 $\mu$g of plasmid pL133 DNA was dissolved in 2 $\mu$l of 10X HindIII buffer and 16 $\mu$l of H$_2$O. About 1 $\mu$l (~10 units) of restriction enzyme HindIII was added to the solution of DNA, and the resulting reaction was incubated at 37°C for 2 hours. The DNA was then diluted to 100 $\mu$l with TE buffer and treated with calf-intestinal alkaline phosphatase in substantial accordance with the procedure in Example 9. The HindIII-digested plasmid pL133 DNA was extracted twice with phenol and once with chloroform, precipitated with ethanol, and resuspended in 10 $\mu$l of TE buffer.

About 5 $\mu$l of the ~0.87 Kb HindIII restriction fragment of plasmid pBLcat were added to the 1.5 $\mu$l of HindIII-digested plasmid pL133, and then, 1 $\mu$l of 10X ligase buffer, 1 $\mu$l (~1000 units) of T4 DNA ligase, and 1.5 $\mu$l of H$_2$O were added to the solution of DNA, and the resulting reaction was incubated at 16°C overnight. The ligated DNA constituted the desired plasmid pLPC.

The ligated DNA was used to transform E. coli K12 HB101. The transformed cells were plated on L agar containing ampicillin, and the plasmid DNA of the ampicillin-resistant transformants was examined by restriction enzyme analysis to identify the E. coli K12 HB101/pLPC transformants. The ~0.87 Kb HindIII restriction fragment that encodes the BK enhancer and Ad2 late promoter could insert into HindIII-digested plasmid pSBLcat in one of two orientations, only one of which yields plasmid pLPC. A restriction site and function map of plasmid pLPC is presented in Figure 9 of the accompanying drawings.

## Example 14

### Construction of Plasmids pLPC4 and pLPC5

About 1 $\mu$g (1 $\mu$l) of the BK virus DNA prepared in Example 8 and 1 $\mu$g of plasmid pLPC (1 $\mu$l) were dissolved in 2 $\mu$l of 10X EcoRI buffer and 14 $\mu$l of H$_2$O. About 2 $\mu$l (~10 units) of restriction enzyme EcoRI were added to the solution of DNA, and the resulting reaction was incubated at 37°C for 2 hours. The EcoRI-digested mixture of BK virus and plasmid pLPC DNA was extracted once with buffered phenol and once with chloroform. Then, the DNA was collected by adjusting the NaCl concentration to 0.25 M, adding two volumes of ethanol, incubating the solution in a dry ice-ethanol bath for 2 minutes, and centrifuging the solution to pellet the DNA. The supernatant was discarded, and the DNA pellet was rinsed with 70% ethanol, dried, and resuspended in 12 $\mu$l of TE buffer.

About 13 $\mu$l of H$_2$O and 3 $\mu$l of 10X ligase buffer were added to the EcoRI-digested mixture of BK virus and plasmid pLPC DNA. Two $\mu$l (~1000 units) of T4 DNA ligase were added to the solution of DNA, and the resulting reaction was incubated at 16°C for 2 hours. The ligated DNA, constituting the desired plasmids pLPC4 and pLPC5, which differ only with respect to the orientation of the inserted BK virus DNA, was used to transform E. coli K12 HB101 competent cells. The transformed cells were plated on L agar containing 100 $\mu$g/ml ampicillin. The E. coli K12 HB101/pLPC4 and E. coli K12 HB101/pLPC5 transformants were identified by their ampicillin-resistant phenotype and by restriction enzyme analysis of their plasmid DNA. A restriction site and function map of plasmid pLPC4 is presented in Figure 10 of the accompanying drawings.

## Example 15

### Construction of Plasmids pLPChyg1 and pLPChyg2

E. coli K12 RR1/pSV2hyg cells are obtained from the NRRL under the accession number NRRL B-18039. Plasmid pSV2hyg DNA is obtained from the cells in substantial accordance with the procedure of Example 10. A restriction site and function map of plasmid pSV2hyg is presented in Figure 11 of the accompanying drawings.

About 10 $\mu$g (in 10 $\mu$l of TE buffer) of plasmid pSV2hyg were added to 2 $\mu$l of 10X BamHI buffer and 6 $\mu$l of H$_2$O. About 2 $\mu$l (about 20 units) of restriction enzyme BamHI were added to the solution of DNA, and the resulting reaction was incubated at 37°C for 2 hours. The reaction was extracted first with phenol and then was extracted twice with chloroform. The BamHI-digested plasmid pSV2hyg DNA was loaded onto an agarose gel to isolate the ~2.5 Kb restriction fragment containing the hygromycin resistance gene.

About 5 $\mu$l of 10X Klenow buffer (0.2 mM in each of the four dNTPs; 0.5 M Tris-HCl, pH = 7.8; 50 mM MgCl$_2$; 0.1 M 2-mercaptoethanol; and 100 $\mu$g/ml BSA) and 35 $\mu$l of H$_2$O were added to the solution of BamHI-digested plasmid pSV2hyg DNA, and then, about 25 units of Klenow enzyme (about 5 $\mu$l, as marketed by BRL) were added to the mixture of DNA, and the resulting reaction was incubated at 16°C for 30 minutes. The Klenow-treated, BamHI-digested plasmid pSV2hyg DNA was extracted once with phenol and once with chloroform and then precipitated with ethanol. About 2 $\mu$g of the desired fragment were obtained and suspended in 5 $\mu$l of TE buffer.

About 10 $\mu$g (10 $\mu$l) of plasmid pLPC DNA were added to 2 $\mu$l of 10X StuI buffer and 6 $\mu$l of H$_2$O. About 2 $\mu$l (~10 units) of restriction enzyme StuI were added to the solution of DNA, and the resulting reaction was incubated at 37°C for 2 hours. The StuI-digested plasmid pLPC DNA was precipitated with ethanol, collected by centrifugation, and resuspended in 2 $\mu$l of 10X NdeI buffer (1.5 M NaCl; 0.1 M Tris-HCl, pH = 7.8; 70 mM MgCl$_2$; 60 mM 2-mercaptoethanol; and 1 mg/ml BSA) and 16 $\mu$l of H$_2$O. About 2 $\mu$l (~10 units) of restriction enzyme NdeI were added to the solution of StuI-digested DNA, and the resulting reaction was incubated at 37°C for 2 hours.

The NdeI-StuI-digested plasmid pLPC DNA was precipitated with ethanol, collected by centrifugation, and resuspended in 5 $\mu$l of 10X Klenow buffer and 40 $\mu$l of H$_2$O. About 5 $\mu$l (~25 units) of Klenow enzyme were added to the solution of DNA, and the resulting reaction was incubated at 16°C for 30 minutes. After the Klenow reaction, the reaction mixture was loaded onto an agarose gel, and the ~5.82 Kb NdeI-StuI restriction fragment was isolated from the gel. About 5 $\mu$g of the desired fragment were obtained and suspended in 5 $\mu$l of TE buffer.

About 2 $\mu$l of the ~2.5 Kb Klenow-treated BamHI restriction fragment of plasmid pSV2hyg were mixed with about 1 $\mu$l of the ~5.82 Kb Klenow-treated NdeI-StuI restriction fragment of plasmid pLPC, and about 3 $\mu$l of 10X ligase buffer, 2 $\mu$l of T4 DNA ligase (~1000 units), 1 $\mu$l of T4 RNA ligase (~1 unit), and 14 $\mu$l of

$H_2O$ were added to the solution of DNA. The resulting reaction was incubated at 16°C overnight. The ligated DNA constituted the desired plasmids pLPChyg1 and pLPChyg2, which differ only with respect to the orientation of the ~2.5 Kb Klenow-treated, BamHI restriction fragment of plasmid pSV2hyg. A restriction site and function map of plasmid pLPChyg1 is presented in Figure 12 of the accompanying drawings. The ligated DNA was used to transform E. coli K12 HB101 and the desired E. coli K12 HB101/pLPChyg1 and E. coli K12 HB101/pLPChyg2 transformants were plated on L agar containing ampicillin and identified by restriction enzyme analysis of their plasmid DNA.

Example 16

Construction of Plasmids pLPChd1 and pLPChd2

About 20 μg of plasmid pSV2-dhfr (ATCC 37146) in 20 μl of TE buffer is added to 10 μl of 10X BamHI buffer and 60 μl of $H_2O$. About 10 μl (~50 units) of restriction enzyme BamHI are added and the resulting reaction is incubated at 37°C for two hours. The BamHI-digested plasmid DNA is then precipitated with ethanol, collected by centrifugation, and resuspended in 5 μl of 10X Klenow buffer, 45 μl of $H_2O$, and 2 μl (~100 units) of Klenow enzyme. The reaction is incubated at 16°C for 30 minutes and then the reaction mixture is loaded onto an agarose gel and electrophoresed until the digestion products are clearly separated. The ~1.9 Kb Klenow-treated, BamHI restriction fragment that comprises the dhfr gene is isolated from the gel and prepared for ligation in substantial accordance with the procedure of Example 9. About 4 μg of the desired fragment are obtained and suspended in 5 μl of TE buffer.

About 200 μg of plasmid pLPChyg1 in 100 μl of TE buffer were added to 15 μl of 10X EcoRI buffer and 30 μl of $H_2O$. About 5 μl (~50 units) of restriction enzyme EcoRI were added to the solution of plasmid pLPChyg1 DNA, and the resulting reaction was incubated at 37°C for about 10 minutes. The short reaction time was calculated to produce a partial EcoRI digestion. Plasmid pLPChyg1 has two EcoRI restriction sites, one of which is within the coding sequence of the hygromycin resistance-conferring (HmR) gene, and it was desired to insert the dhfr-gene-containing restriction fragment into the EcoRI site of plasmid pLPChyg1 that is not in the HmR gene. The partially-EcoRI-digested plasmid pLPChyg1 DNA was loaded onto an agarose gel and electrophoresed until the singly-cut plasmid pLPChyg1 DNA was separated from uncut plasmid DNA and the other digestion products. The singly-cut DNA was isolated from the gel and prepared for ligation in substantial accordance with the procedure of Example 9. About 2 μg of the singly-EcoRI-cut plasmid pLPChyg1 were obtained and suspended in 25 μl of TE buffer. To this sample, about 5 μl (~25 units) of Klenow enzyme, 5 μl of 10X Klenow buffer, and 40 μl of $H_2O$ were added, and the resulting reaction was incubated at 16°C for 60 minutes. The Klenow-treated, partially-EcoRI-digested DNA was then extracted twice with phenol and then once with chloroform, precipitated with ethanol, and resuspended in 25 μl of TE buffer.

About 5 μl of the ~1.9 Kb Klenow-treated BamHI restriction fragment containing the dhfr gene and about 5 μl of the singly-EcoRI-cut plasmid pLPChyg1 DNA were mixed together, and 1 μl of 10X ligase buffer, 5 μl of $H_2O$, 1 μl (~500 units) of T4 DNA ligase, and 1 μl (~2 units) of T4 RNA ligase were added to the mixture of DNA, and the resulting reaction was incubated at 16°C overnight. The ligated DNA constituted the desired plasmids pLPChd1 and pLPChd2, which differ only with respect to the orientation of the ~1.9 Kb fragment that comprises the dhfr gene.

The ligated DNA was used to transform competent E. coli K12 HB101 cells. The transformed cells were plated onto L agar containing 100 μg/ml ampicillin, and the ampicillin-resistant transformants were analyzed by restriction enzyme analysis of their plasmid DNA to identify the E. coli K12 HB101/pLPChd1 and E. coli K12 HB101/pLPChd2 transformants. A restriction site and function map of plasmid pLPChd1 is presented in Figure 13 of the accompanying drawings. Plasmid pLPChd1 and plasmid pLPChd2 DNA were subsequently isolated from the appropriate transformants.

Example 17

Construction of Plasmid phd

To construct plasmid phd, it was necessary to prepare the plasmid pLPChd1 DNA, used as starting material in the construction of plasmid phd, from E. coli host cells that lack an adenine methylase, such as that encoded by the dam gene, the product of which methylates the adenine residue in the sequence 5'-GATC-3'. E. coli K12 GM48 (NRRL B-15725) lacks a functional dam methylase and so is a suitable host to use for the purpose of preparing plasmid pLPChd1 DNA for use as starting material in the construction of

29

plasmid phd.

E. coli K12 GM48 cells were cultured and made competent for transformation, and plasmid pLPChyg1 was used to transform the E. coli K12 GM48 cells. The transformed cells were plated on L agar containing ampicillin, and once the ampicillin-resistant, E. coli K12 GM48/pLPChd1 transformants had formed colonies, one such colony was used to prepare plasmid pLPChd1 DNA. About 1 mg of plasmid pLPChd1 DNA was obtained and suspended in about 1 ml of TE buffer.

About 2 $\mu$g of plasmid pLPChd1 DNA in 2 $\mu$l of TE buffer were added to 2 $\mu$l of 10X BclI buffer (750 mM KCl; 60 mM Tris-HCl, pH = 7.4; 100 mM MgCl$_2$; 10 mM DTT and 1 mg/ml BSA) and 14 $\mu$l of H$_2$O. About 2 $\mu$l (~10 units) of restriction enzyme BclI were added to the solution of plasmid pLPChd1 DNA, and the resulting reaction was incubated at 50°C for two hours. The reaction was stopped by extracting the mixture once with phenol and twice with chloroform.

About 1 $\mu$l of the BclI-digested plasmid pLPChd1 DNA was added to 1 $\mu$l of 10X ligase buffer, 8 $\mu$l of H$_2$O and 1 $\mu$l (~500 units) of T4 DNA ligase. The ligation reaction was incubated at 16°C overnight, and the ligated DNA constituted the desired plasmid phd. Plasmid phd results from the deletion of the extra BclI linkers that attached during the construction of plasmid pLPcat and the two adjacent BclI restriction fragments of a total size of about 1.45 Kb from plasmid pLPChd1. A restriction site and function map of plasmid phd is presented in Figure 14 of the accompanying drawings. Plasmid phd facilitates the expression of any DNA sequence from the BK virus enhancer-adenovirus late promoter of the present invention, because the DNA to be expressed, such as human protein S, can be readily inserted in the correct position for expression at the single BclI site on plasmid phd.

The ligated DNA was used to transform E. coli K12 GM48 and the transformed cells were plated on L-agar containing ampicillin. The ampicillin-resistant E. coli K12 GM48/phd transformants were identified by restriction enzyme analysis of their plasmid DNA.

Example 18

Construction of Human Protein S Expression Vectors

A. Construction of Plasmid pShd

About 2 $\mu$g of plasmid phd DNA in 2 $\mu$l of TE buffer were digested with BclI restriction enzyme in substantial accordance with the teaching of Example 17. The BclI-digested plasmid DNA was precipitated with ethanol, collected by centrifugation, and resuspended in 5 $\mu$l of 10X Klenow buffer and 40 $\mu$l of H$_2$O. About 5 $\mu$l (~25 units) of Klenow enzyme were added to the solution of DNA and the resulting reaction was incubated at 16°C for 30 minutes. After the Klenow reaction, the reaction mixture was loaded onto an agarose gel and the vector fragment was isolated from the gel. About 0.5 $\mu$g of the desired fragment were obtained and suspended in 10 $\mu$l of TE buffer.

Approximately 20 $\mu$g of plasmid pHHS-IIa DNA were dissolved in 10 $\mu$l of 10X FnuDII reaction buffer, 10 $\mu$l (~20 units) of restriction enzyme FnuDII, and 80 $\mu$l of H$_2$O, and the reaction mixture was placed at 37°C for two hours. The reaction was adjusted by adding NaCl to 50 mM and 5 $\mu$l (~50 units) of restriction enzyme EcoRV. The resulting reaction was incubated at 37°C for two hours. After the EcoRV digestion, the reaction mixture was loaded onto a 1.0% agarose gel to isolate the desired ~2.7 Kb FnuDII-EcoRV restriction fragment. Approximately 10 $\mu$g of the fragment were obtained and suspended in 50 $\mu$l of TE buffer.

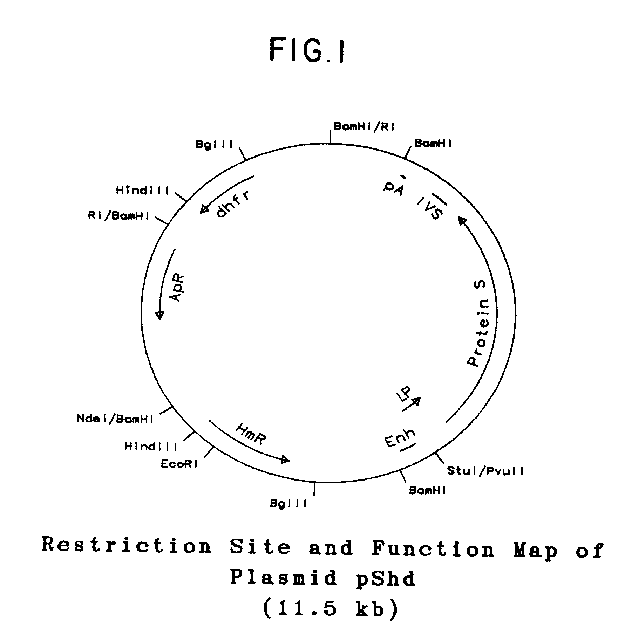

About 5 $\mu$l of the BclI-digested plasmid phd were ligated to about 2 $\mu$l of the ~2.7 Kb FnuDII-EcoRV restriction fragment in substantial accordance with the teaching of preceding examples except that ~0.4 units of RNA ligase (BRL) was added to the ligation reaction. The ligated DNA constituted the desired plasmid pShd; a restriction site and function map of the plasmid is presented in Figure 1 of the accompanying drawings.

E. coli K12 HB101 cells were made competent for transformation and the ligated DNA prepared above was used to transform the cells. Aliquots of the transformation mix were plated on L-agar containing ampicillin. The ampicillin-resistant E. coli K12 HB101/pShd transformants were identified by restriction enzyme analysis.

Alternatively, should one wish to isolate a fragment that contains only the protein S coding sequence, the additional base pairs can be removed from the 5' end of the molecule to produce a molecule that begins with the ATG start codon. Thus, linear DNA can be digested with an exonuclease called BAL 31 nuclease that attacks both ends of the molecule and progressively shortens the molecule at a predictable rate. Accordingly, five $\mu$g of pHHS-IIa are digested with 5 units of FnuDII in 100 $\mu$l of 6 mM NaCl, 6 mM Tris-HCl (pH = 7.4), 6 $\mu$M MgCl$_2$, and 6 $\mu$M 2-mercaptoethanol. Following an ethanol precipitation, the DNA fragments are separated by electrophoresis on a 3.5% polyacrylamide gel and the 2.7 Kb fragment is isolated as described in Example 3. The fragment is now prepared for digestion with BAL 31. The reaction conditions are as follows: 5 $\mu$g of DNA is dissolved in 50 $\mu$l of 0.5 M NaCl, 12.5 mM CaCl$_2$, 12.5 mM MgCl$_2$, 20 mM Tris-HCl, pH = 8.0, and 1 mM EDTA, pH = 8.0. One unit of BAL 31 is added and the reaction is allowed to continue at 30 °C for 30 seconds. Following an ethanol precipitation, the DNA is now treated with T4 DNA polymerase to fill in any staggered ends that may have been left by the action of the BAL 31. The DNA is resuspended in 19 $\mu$l of reaction buffer (16.6 mM (NH$_4$)$_2$SO$_4$, 0.67 M Tris-HCl, pH = 8.8, 0.67 mM MgCl$_2$, 1 M 2-mercaptoethanol, and 6.7 $\mu$M EDTA). To this is added 1 $\mu$l of T4 DNA polymerase (0.5 units) diluted 1 to 10 in 50 mM Tris-HCl, pH = 7.5, 100 mM (NH$_4$)$_2$SO$_4$, 1 mg/ml BSA and 10 mM 2-mercaptoethanol. The reaction is incubated for 10 minutes at 37 °C before the addition of 1 $\mu$l of a dNTP mix containing 2 mM of each dNTP. The reaction continues at 37 °C for an additional 10 minutes followed by an ethanol precipitation. This fragment is now ready to ligate to any blunt-end vector. Under these conditions BAL 31 removes about 25 base pairs/minute/end. After ligation, transformation into an E. coli host and subsequent isolation of DNA from independent clones, one can use DNA sequencing to determine which clone contains the exact sequence desired.

The plasmid pHHS-IIa also contains additional non-coding DNA sequences at the 3' end, including the natural stop codon TAA which immediately follows the last nucleotide triplet of the coding sequence. These sequences can be removed as follows: 5 $\mu$g of the intact plasmid are digested to completion in 100 $\mu$l of 1X FnuDII reaction buffer containing 5 units of FnudII. The DNA is precipitated in ethanol and recovered by centrifugation. The pellet is dried and resuspended in 100 $\mu$l of 1X EcoRI reaction buffer containing 5 units of EcoRI.

After 90 minutes at 37 °C, the DNA is again precipitated in ethanol and is applied to a 3.5% polyacrylamide gel. The products are separated by electrophoresis and the ~2030 base pair band is extracted as described in Example 3. Since the EcoRI site is located only one base away from the natural stop codon, and digestion with EcoRI leaves only the initial G residue, it is necessary to replace the remaining bases of the EcoRI site, the T residue and the stop codon. Thus, a linker has been designed for this purpose and can be synthesized by any of the standard techniques:

```
5'-AATTCTTAATAG-3'
   |||||||||
3'-GAATTATCCTAG-5'
```

This linker includes the natural sequence and incorporates an additional stop codon in frame to insure termination at that point. In addition, this linker contains a CTAG overhang that is compatable with BamHI, BglII or BclI ends. In a three-piece ligation reaction these fragments can be ligated to any vector that contains one blunt end along with a BamHI, BglII or BclI end.

B Transformation

The transformation procedure described below refers to 293 cells as the host cell line; however, the procedure is generally applicable to most eukaryotic cell lines. 293 cells were obtained from the ATCC under the accession number CRL 1573 in a 25 mm$^2$ flask containing a confluent monolayer of about 5.5 x 10$^6$ cells in Eagle's Minimum Essential Medium with 10% heat-inactivated horse serum. The flask was incubated at 37 °C; medium was changed twice weekly. The cells were sub-cultured by removing the medium, rinsing with Hank's Balanced Salts solution (Gibco), adding 0.25% trypsin for 1-2 minutes, rinsing with fresh medium, aspirating, and dispensing into new flasks at a subcultivation ratio of 1:5 or 1:10.

One day prior to transformation, cells were seeded at 0.7 x 10$^6$ cells per dish. The medium was changed 4 hours prior to transformation. Sterile, ethanol-precipitated plasmid DNA dissolved in TE buffer was used to prepare a 2X DNA-CaCl$_2$ solution containing 40 $\mu$g/ml DNA and 250 mM CaCl$_2$. 2X HBS was prepared containing 280 mM NaCl, 50 mM Hepes, and 1.5 mM sodium phosphate, with the pH adjusted to 7.05-7.15. The 2X DNA-CaCl$_2$ solution was added dropwise to an equal volume of sterile 2X HBS. A one ml sterile plastic pipette with a cotton plug was inserted into the mixing tube that contains the 2X HBS, and bubbles were introduced by blowing while the DNA was being added. The calcium-phosphate-DNA precipitate was allowed to form without agitation for 30-45 minutes at room temperature.

The precipitate was then mixed by gentle pipetting with a plastic pipette, and one ml (per plate) of precipitate was added directly to the 10 ml of growth medium that covers the recipient cells. After 4 hours of incubation at 37°C, the medium was replaced with DMEM with 10% fetal bovine serum and the cells allowed to incubate for an additional 72 hours before providing selective pressure. For transformants expressing recombinant human protein S, the growth medium contains 1 to 10 μg/ml vitamin K, a cofactor required for γ-carboxylation of the protein.

Hygromycin is added to the growth medium to a final concentration of about 200 μg/ml. The cells are then incubated at 37°C for 2-4 weeks with medium changes at 3 to 4 day intervals. The resulting hygromycin-resistant colonies are transferred to individual culture flasks for characterization. 293 cells are dhfr positive, so 293 transformants that contain plasmids comprising the dhfr gene are not selected solely on the basis of the dhfr-positive phenotype, which is the ability to grow in media that lacks hypoxanthine and thymine. Cell lines that do lack a functional dhfr gene and are transformed with dhfr-containing plasmids can be selected for on the basis of the dhfr + phenotype.

The use of the dihydrofolate reductase gene as a selectable marker for introducing a gene or plasmid into a dhfr-deficient cell line and the subsequent use of methotrexate to amplify the copy number of the plasmid has been well established in the literature. Although the use of dhfr as a selectable and amplifiable marker in dhfr-producing cells has not been well studied, evidence in the literature would suggest that dhfr can be used as a selectable marker in dhfr-producing cells and for gene amplification. The use of the present invention is not limited by the selectable marker used. Moreover, amplifiable markers such as metallothionein genes, adenosine deaminase genes, or members of the multigene resistance family, exemplified by P-glycoprotein, can be utilized.

Although the foregoing invention has been described in some detail by way of illustration and examples for purposes of clarity and understanding, it will be obvious that certain changes and modifications may be practiced within the scope of the appended claims.

**Claims**

1. A double-stranded deoxyribonucleic acid that encodes human protein, wherein the coding strand is:

```
                                              5'-R'-GCA AAT TCT
    TTA CTT GAA GAA ACC AAA CAG GGT AAT CTT GAA AGA GAA TGC ATC GAA
    GAA CTG TGC AAT AAA GAA GAA GCC AGG GAG GTC TTT GAA AAT GAC CCG
    GAA ACG GAT TAT TTT TAT CCA AAA TAC TTA GTT TGT CTT CGC TCT TTT
    CAA ACT GGG TTA TTC ACT GCT GCA CGT CAG TCA ACT AAT GCT TAT CCT
    GAC CTA AGA AGC TGT GTC AAT GCC ATT CCA GAC CAG TGT AGT CCT CTG
```

```
CCA TGC AAT GAA GAT GGA TAT ATG AGC TGC AAA GAT GGA AAA GCT TCT
TTT ACT TGC ACT TGT AAA CCA GGT TGG CAA GGA GAA AAG TGT GAA TTT
GAC ATA AAT GAA TGC AAA GAT CCC TCA AAT ATA AAT GGA GGT TGC AGT
CAA ATT TGT GAT AAT ACA CCT GGA AGT TAC CAC TGT TCC TGT AAA AAT
GGT TTT GTT ATG CTT TCA AAT AAG AAA GAT TGT AAA GAT GTG GAT GAA
TGC TCT TTG AAG CCA AGC ATT TGT GGC ACA GCT GTG TGC AAG AAC ATC
CCA GGA GAT TTT GAA TGT GAA TGC CCC GAA GGC TAC AGA TAT AAT CTC
AAA TCA AAG TCT TGT GAA GAT ATA GAT GAA TGC TCT GAG AAC ATG TGT
GCT CAG CTT TGT GTC AAT TAC CCT GGA GGT TAC ACT TGC TAT TGT GAT
GGG AAG AAA GGA TTC AAA CTT GCC CAA GAT CAG AAG AGT TGT GAG GTT
GTT TCA GTG TGC CTT CCC TTG AAC CTT GAC ACA AAG TAT GAA TTA CTT
TAC TTG GCG GAG CAG TTT GCA GGG GTT GTT TTA TAT TTA AAA TTT CGT
TTG CCA GAA ATC AGC AGA TTT TCA GCA GAA TTT GAT TTC CGG ACA TAT
GAT TCA GAA GGC GTG ATA CTG TAC GCA GAA TCT ATC GAT CAC TCA GCG
TGG CTC CTG ATT GCA CTT CGT GGT GGA AAG ATT GAA GTT CAG CTT AAG
AAT GAA CAT ACA TCC AAA ATC ACA ACT GGA GGT GAT GTT ATT AAT AAT
GGT CTA TGG AAT ATG GTG TCT GTG GAA GAA TTA GAA CAT AGT ATT AGC
ATT AAA ATA GCT AAA GAA GCT GTG ATG GAT ATA AAT AAA CCT GGA CCC
CTT TTT AAG CCG GAA AAT GGA TTG CTG GAA ACC AAA GTA TAC TTT GCA
GGA TTC CCT CGG AAA GTG GAA AGT GAA CTC ATT AAA CCG ATT AAC CCT
CGT CTA GAT GGA TGT ATA CGA AGC TGG AAT TTG ATG AAG CAA GGA GCT
TCT GGA ATA AAG GAA ATT ATT CAA GAA AAA CAA AAT AAG CAT TGC CTG
GTT ACT GTG GAG AAG GGC TCC TAC TAT CCT GGT TCT GGA ATT GCT CAA
TTT CAC ATA GAT TAT AAT AAT GTA TCC AGT GCT GAG GGT TGG CAT GTA
AAT GTG ACC TTG AAT ATT CGT CCA TCC ACG GGC ACT GGT GTT ATG CTT
GCC TTG GTT TCT GGT AAC AAC ACA GTG CCC TTT GCT GTG TCC TTG GTG
GAC TCC ACC TCT GAA AAA TCA CAG GAT ATT CTG TTA TCT GTT GAA AAT
ACT GTA ATA TAT CGG ATA CAG GCC CTA AGT CTA TGT TCC GAT CAA CAA
TCT CAT CTG GAA TTT AGA GTC AAC AGA AAC AAT CTG GAG TTG TCG ACA
CCA CTT AAA ATA GAA ACC ATC TCC CAT GAA GAC CTT CAA AGA CAA CTT
GCC GTC TTG GAC AAA GCA ATG AAA GCA AAA GTG GCC ACA TAC CTG GGT
```

```
GGC CTT CCA GAT GTT CCA TTC AGT GCC ACA CCA GTG AAT GCC TTT TAT
AAT GGC TGC ATG GAA GTG AAT ATT AAT GGT GTA CAG TTG GAT CTG GAT
GAA GCC ATT TCT AAA CAT AAT GAT ATT AGA GCT CAC TCA TGT CCA TCA
GTT TGG AAA AAG ACA AAG AAT TCT-3'
```

33

wherein
R' is

5'-ATG AGG GTC CTG GGT GGG CGC TGC GGG GCG CCG CTG

GCG TGT CTC CTC CTA GTG CTT CCC GTC TCA GAG GCA AAC CTT CTG TCA

AAG CAA CAG GCT TCA CAA GTC CTG GTT AGG AAG CGT CGT-3',

A is deoxyadenyl,
G is deoxyguanyl,
C is deoxycytidyl, and
T is thymidyl.

**2.** A double-stranded deoxyribonucleic acid that encodes a polypeptide with human protein S activity, wherein the coding strand is:

5'-GCA AAT TCT

TTA CTT GAA GAA ACC AAA CAG GGT AAT CTT GAA AGA GAA TGC ATC GAA

GAA CTG TGC AAT AAA GAA GAA GCC AGG GAG GTC TTT GAA AAT GAC CCG

GAA ACG GAT TAT TTT TAT CCA AAA TAC TTA GTT TGT CTT CGC TCT TTT

CAA ACT GGG TTA TTC ACT GCT GCA CGT CAG TCA ACT AAT GCT TAT CCT

GAC CTA AGA AGC TGT GTC AAT GCC ATT CCA GAC CAG TGT AGT CCT CTG

CCA TGC AAT GAA GAT GGA TAT ATG AGC TGC AAA GAT GGA AAA GCT TCT

TTT ACT TGC ACT TGT AAA CCA GGT TGG CAA GGA GAA AAG TGT GAA TTT

GAC ATA AAT GAA TGC AAA GAT CCC TCA AAT ATA AAT GGA GGT TGC AGT

CAA ATT TGT GAT AAT ACA CCT GGA AGT TAC CAC TGT TCC TGT AAA AAT

GGT TTT GTT ATG CTT TCA AAT AAG AAA GAT TGT AAA GAT GTG GAT GAA

TGC TCT TTG AAG CCA AGC ATT TGT GGC ACA GCT GTG TGC AAG AAC ATC

CCA GGA GAT TTT GAA TGT GAA TGC CCC GAA GGC TAC AGA TAT AAT CTC

AAA TCA AAG TCT TGT GAA GAT ATA GAT GAA TGC TCT GAG AAC ATG TGT

GCT CAG CTT TGT GTC AAT TAC CCT GGA GGT TAC ACT TGC TAT TGT GAT

34

```
GGG AAG AAA GGA TTC AAA CTT GCC CAA GAT CAG AAG AGT TGT GAG GTT
GTT TCA GTG TGC CTT CCC TTG AAC CTT GAC ACA AAG TAT GAA TTA CTT
TAC TTG GCG GAG CAG TTT GCA GGG GTT GTT TTA TAT TTA AAA TTT CGT
TTG CCA GAA ATC AGC AGA TTT TCA GCA GAA TTT GAT TTC CGG ACA TAT
GAT TCA GAA GGC GTG ATA CTG TAC GCA GAA TCT ATC GAT CAC TCA GCG
TGG CTC CTG ATT GCA CTT CGT GGT GGA AAG ATT GAA GTT CAG CTT AAG
AAT GAA CAT ACA TCC AAA ATC ACA ACT GGA GGT GAT GTT ATT AAT AAT
GGT CTA TGG AAT ATG GTG TCT GTG GAA GAA TTA GAA CAT AGT ATT AGC
ATT AAA ATA GCT AAA GAA GCT GTG ATG GAT ATA AAT AAA CCT GGA CCC
CTT TTT AAG CCG GAA AAT GGA TTG CTG GAA ACC AAA GTA TAC TTT GCA
GGA TTC CCT CGG AAA GTG GAA AGT GAA CTC ATT AAA CCG ATT AAC CCT
CGT CTA GAT GGA TGT ATA CGA AGC TGG AAT TTG ATG AAG CAA GGA GCT
TCT GGA ATA AAG GAA ATT ATT CAA GAA AAA CAA AAT AAG CAT TGC CTG
GTT ACT GTG GAG AAG GGC TCC TAC TAT CCT GGT TCT GGA ATT GCT CAA
TTT CAC ATA GAT TAT AAT AAT GTA TCC AGT GCT GAG GGT TGG CAT GTA
AAT GTG ACC TTG AAT ATT CGT CCA TCC ACG GGC ACT GGT GTT ATG CTT
GCC TTG GTT TCT GGT AAC AAC ACA GTG CCC TTT GCT GTG TCC TTG GTG
GAC TCC ACC TCT GAA AAA TCA CAG GAT ATT CTG TTA TCT GTT GAA AAT
ACT GTA ATA TAT CGG ATA CAG GCC CTA AGT CTA TGT TCC GAT CAA CAA
TCT CAT CTG GAA TTT AGA GTC AAC AGA AAC AAT CTG GAG TTG TCG ACA
CCA CTT AAA ATA GAA ACC ATC TCC CAT GAA GAC CTT CAA AGA CAA CTT
GCC GTC TTG GAC AAA GCA ATG AAA GCA AAA GTG GCC ACA TAC CTG GGT
GGC CTT CCA GAT GTT CCA TTC AGT GCC ACA CCA GTG AAT GCC TTT TAT
AAT GGC TGC ATG GAA GTG AAT ATT AAT GGT GTA CAG TTG GAT CTG GAT
GAA GCC ATT TCT AAA CAT AAT GAT ATT AGA GCT CAC TCA TGT CCA TCA
GTT TGG AAA AAG ACA AAG AAT TCT-3'
```

wherein
    A is deoxyadenyl,
    G is deoxyguanyl,
    C is deoxycytidyl, and
    T is thymidyl.

3.    A recombinant DNA vector which comprises the double-stranded deoxyribonucleic acid of Claim 1 or 2.

4.    The vector of Claim 3 which is plasmid pShd produced by ligating the ~2.7 kb protein S-containing Fnu DII-EcoRV restriction fragment from plasmid pHHS-IIa (NRRL B-18071) to Bcl I-digested Klenow-treated plasmid phd (as shown in Figure 14).

5.    The vector of Claim 3 which is plasmid pHHS-IIa (NRRL B-18071).

6.    Escherichia coli cells transformed with the vector of Claim 3, 4 or 5.

7.    A culture of cells transformed with the vector of Claim 3 or 4.

**8.** The culture of Claim 7 wherein the cells are human kidney 293 cells.

**9.** A process for producing human protein S which comprises culturing the culture of Claim 7 or 8 under conditions permitting expression of human protein S and recovering the expressed protein.

**10.** Human protein S when produced by the process of Claim 9.

**11.** A pharmaceutical composition comprising human protein S produced by the process of Claim 9 in admixture with a pharmaceutically acceptable carrier.

**12.** The composition of Claim 11 which is suitable for parenteral administration.

**Patentansprüche**

**1.** Doppelsträngige Desoxyribonukleinsäure, die humanes Protein S kodiert, wobei der kodierende Strang folgender ist:

```
                                                      5'-R'-GCA AAT TCT
TTA CTT GAA GAA ACC AAA CAG GGT AAT CTT GAA AGA GAA TGC ATC GAA
GAA CTG TGC AAT AAA GAA GAA GCC AGG GAG GTC TTT GAA AAT GAC CCG
GAA ACG GAT TAT TTT TAT CCA AAA TAC TTA GTT TGT CTT CGC TCT TTT
CAA ACT GGG TTA TTC ACT GCT GCA CGT CAG TCA ACT AAT GCT TAT CCT
GAC CTA AGA AGC TGT GTC AAT GCC ATT CCA GAC CAG TGT AGT CCT CTG
CCA TGC AAT GAA GAT GGA TAT ATG AGC TGC AAA GAT GGA AAA GCT TCT
TTT ACT TGC ACT TGT AAA CCA GGT TGG CAA GGA GAA AAG TGT GAA TTT
GAC ATA AAT GAA TGC AAA GAT CCC TCA AAT ATA AAT GGA GGT TGC AGT
CAA ATT TGT GAT AAT ACA CCT GGA AGT TAC CAC TGT TCC TGT AAA AAT
GGT TTT GTT ATG CTT TCA AAT AAG AAA GAT TGT AAA GAT GTG GAT GAA
TGC TCT TTG AAG CCA AGC ATT TGT GGC ACA GCT GTG TGC AAG AAC ATC
CCA GGA GAT TTT GAA TGT GAA TGC CCC GAA GGC TAC AGA TAT AAT CTC
AAA TCA AAG TCT TGT GAA GAT ATA GAT GAA TGC TCT GAG AAC ATG TGT
GCT CAG CTT TGT GTC AAT TAC CCT GGA GGT TAC ACT TGC TAT TGT GAT
GGG AAG AAA GGA TTC AAA CTT GCC CAA GAT CAG AAG AGT TGT GAG GTT
GTT TCA GTG TGC CTT CCC TTG AAC CTT GAC ACA AAG TAT GAA TTA CTT
TAC TTG GCG GAG CAG TTT GCA GGG GTT GTT TTA TAT TTA AAA TTT CGT
TTG CCA GAA ATC AGC AGA TTT TCA GCA GAA TTT GAT TTC CGG ACA TAT
GAT TCA GAA GGC GTG ATA CTG TAC GCA GAA TCT ATC GAT CAC TCA GCG
TGG CTC CTG ATT GCA CTT CGT GGT GGA AAG ATT GAA GTT CAG CTT AAG
AAT GAA CAT ACA TCC AAA ATC ACA ACT GGA GGT GAT GTT ATT AAT AAT
GGT CTA TGG AAT ATG GTG TCT GTG GAA GAA TTA GAA CAT AGT ATT AGC
ATT AAA ATA GCT AAA GAA GCT GTG ATG GAT ATA AAT AAA CCT GGA CCC
CTT TTT AAG CCG GAA AAT GGA TTG CTG GAA ACC AAA GTA TAC TTT GCA
GGA TTC CCT CGG AAA GTG GAA AGT GAA CTC ATT AAA CCG ATT AAC CCT
CGT CTA GAT GGA TGT ATA CGA AGC TGG AAT TTG ATG AAG CAA GGA GCT
TCT GGA ATA AAG GAA ATT ATT CAA GAA AAA CAA AAT AAG CAT TGC CTG
GTT ACT GTG GAG AAG GGC TCC TAC TAT CCT GGT TCT GGA ATT GCT CAA
TTT CAC ATA GAT TAT AAT AAT GTA TCC AGT GCT GAG GGT TGG CAT GTA
AAT GTG ACC TTG AAT ATT CGT CCA TCC ACG GGC ACT GGT GTT ATG CTT
GCC TTG GTT TCT GGT AAC AAC ACA GTG CCC TTT GCT GTG TCC TTG GTG
GAC TCC ACC TCT GAA AAA TCA CAG GAT ATT CTG TTA TCT GTT GAA AAT
```

```
ACT GTA ATA TAT CGG ATA CAG GCC CTA AGT CTA TGT TCC GAT CAA CAA

TCT CAT CTG GAA TTT AGA GTC AAC AGA AAC AAT CTG GAG TTG TCG ACA

CCA CTT AAA ATA GAA ACC ATC TCC CAT GAA GAC CTT CAA AGA CAA CTT

GCC GTC TTG GAC AAA GCA ATG AAA GCA AAA GTG GCC ACA TAC CTG GGT

GGC CTT CCA GAT GTT CCA TTC AGT GCC ACA CCA GTG AAT GCC TTT TAT

AAT GGC TGC ATG GAA GTG AAT ATT AAT GGT GTA CAG TTG GAT CTG GAT

GAA GCC ATT TCT AAA CAT AAT GAT ATT AGA GCT CAC TCA TGT CCA TCA

GTT TGG AAA AAG ACA AAG AAT TCT-3'
```

worin R' für
  R' is

```
            5'-ATG AGG GTC CTG GGT GGG CGC TGC GGG GCG CCG CTG

GCG TGT CTC CTC CTA GTG CTT CCC GTC TCA GAG GCA AAC CTT CTG TCA

AAG CAA CAG GCT TCA CAA GTC CTG GTT AGG AAG CGT CGT-3',
```

steht,
A für Desoxyadenyl steht,
G für Desoxyguanyl steht,
C für Desoxycytidyl steht und
T für Thymidyl steht.

2. Doppelsträngige Desoxyribonukleinsäure, die ein Polypeptid mit humaner Protein S Aktivität kodiert, wobei der kodierende Strang folgender ist:

```
                                                      5'-GCA AAT TCT

TTA CTT GAA GAA ACC AAA CAG GGT AAT CTT GAA AGA GAA TGC ATC GAA

GAA CTG TGC AAT AAA GAA GAA GCC AGG GAG GTC TTT GAA AAT GAC CCG

GAA ACG GAT TAT TTT TAT CCA AAA TAC TTA GTT TGT CTT CGC TCT TTT

CAA ACT GGG TTA TTC ACT GCT GCA CGT CAG TCA ACT AAT GCT TAT CCT

GAC CTA AGA AGC TGT GTC AAT GCC ATT CCA GAC CAG TGT AGT CCT CTG

CCA TGC AAT GAA GAT GGA TAT ATG AGC TGC AAA GAT GGA AAA GCT TCT

TTT ACT TGC ACT TGT AAA CCA GGT TGG CAA GGA GAA AAG TGT GAA TTT

GAC ATA AAT GAA TGC AAA GAT CCC TCA AAT ATA AAT GGA GGT TGC AGT

CAA ATT TGT GAT AAT ACA CCT GGA AGT TAC CAC TGT TCC TGT AAA AAT

GGT TTT GTT ATG CTT TCA AAT AAG AAA GAT TGT AAA GAT GTG GAT GAA

TGC TCT TTG AAG CCA AGC ATT TGT GGC ACA GCT GTG TGC AAG AAC ATC

CCA GGA GAT TTT GAA TGT GAA TGC CCC GAA GGC TAC AGA TAT AAT CTC

AAA TCA AAG TCT TGT GAA GAT ATA GAT GAA TGC TCT GAG AAC ATG TGT

GCT CAG CTT TGT GTC AAT TAC CCT GGA GGT TAC ACT TGC TAT TGT GAT
```

```
GGG AAG AAA GGA TTC AAA CTT GCC CAA GAT CAG AAG AGT TGT GAG GTT

GTT TCA GTG TGC CTT CCC TTG AAC CTT GAC ACA AAG TAT GAA TTA CTT

TAC TTG GCG GAG CAG TTT GCA GGG GTT GTT TTA TAT TTA AAA TTT CGT

TTG CCA GAA ATC AGC AGA TTT TCA GCA GAA TTT GAT TTC CGG ACA TAT

GAT TCA GAA GGC GTG ATA CTG TAC GCA GAA TCT ATC GAT CAC TCA GCG

TGG CTC CTG ATT GCA CTT CGT GGT GGA AAG ATT GAA GTT CAG CTT AAG

AAT GAA CAT ACA TCC AAA ATC ACA ACT GGA GGT GAT GTT ATT AAT AAT

GGT CTA TGG AAT ATG GTG TCT GTG GAA GAA TTA GAA CAT AGT ATT AGC

ATT AAA ATA GCT AAA GAA GCT GTG ATG GAT ATA AAT AAA CCT GGA CCC

CTT TTT AAG CCG GAA AAT GGA TTG CTG GAA ACC AAA GTA TAC TTT GCA

GGA TTC CCT CGG AAA GTG GAA AGT GAA CTC ATT AAA CCG ATT AAC CCT

CGT CTA GAT GGA TGT ATA CGA AGC TGG AAT TTG ATG AAG CAA GGA GCT

TCT GGA ATA AAG GAA ATT ATT CAA GAA AAA CAA AAT AAG CAT TGC CTG

GTT ACT GTG GAG AAG GGC TCC TAC TAT CCT GGT TCT GGA ATT GCT CAA

TTT CAC ATA GAT TAT AAT AAT GTA TCC AGT GCT GAG GGT TGG CAT GTA

AAT GTG ACC TTG AAT ATT CGT CCA TCC ACG GGC ACT GGT GTT ATG CTT

GCC TTG GTT TCT GGT AAC AAC ACA GTG CCC TTT GCT GTG TCC TTG GTG

GAC TCC ACC TCT GAA AAA TCA CAG GAT ATT CTG TTA TCT GTT GAA AAT

ACT GTA ATA TAT CGG ATA CAG GCC CTA AGT CTA TGT TCC GAT CAA CAA

TCT CAT CTG GAA TTT AGA GTC AAC AGA AAC AAT CTG GAG TTG TCG ACA

CCA CTT AAA ATA GAA ACC ATC TCC CAT GAA GAC CTT CAA AGA CAA CTT

GCC GTC TTG GAC AAA GCA ATG AAA GCA AAA GTG GCC ACA TAC CTG GGT

GGC CTT CCA GAT GTT CCA TTC AGT GCC ACA CCA GTG AAT GCC TTT TAT

AAT GGC TGC ATG GAA GTG AAT ATT AAT GGT GTA CAG TTG GAT CTG GAT

GAA GCC ATT TCT AAA CAT AAT GAT ATT AGA GCT CAC TCA TGT CCA TCA

GTT TGG AAA AAG ACA AAG AAT TCT-3'
```

worin
A für Desoxyadenyl steht,
G für Desoxyguanyl steht,
C für Desoxycytidyl steht und
T für Thymidyl steht.

3. Rekombinanter DNA Vektor, der die doppelsträngige Desoxyribonukleinsäure nach Anspruch 1 oder 2 enthält.

4. Vektor nach Anspruch 3, welcher Plasmid pShd ist, hergestellt durch Ligation des ~2,7 kb Protein S-enthaltenden FnuDII-EcoRV Restriktionsfragments von Plasmid pHHS-IIa (NRRL B-18071) mit dem BcII-verdauten Klenow-behandelten Plasmid phd (wie in Figur 14 gezeigt).

5. Vektor nach Anspruch 3, welcher Plasmid pHHS-IIa (NRRL B-18071) ist.

6. Escherichia coli Zellen, die mit dem Vektor nach Anspruch 3, 4 oder 5 transformiert sind.

**7.** Zellkultur, die mit dem Vektor nach Anspruch 3 oder 4 transformiert ist.

**8.** Kultur nach Anspruch 7, worin die Zellen humane Nierenzellen 293 sind.

**9.** Verfahren zur Herstellung von humanem Protein S, gekennzeichnet durch Kultivieren der Kultur nach Anspruch 7 oder 8 unter Bedingungen, die die Expression von humanem Protein S und die Gewinnung des exprimierten Proteins erlauben.

**10.** Durch das Verfahren nach Anspruch 9 hergestelltes Protein S.

**11.** Pharmazeutische Zusammensetzung, die durch das Verfahren nach Anspruch 9 hergestelltes humanes Protein S im Gemisch mit einem pharmazeutisch annehmbaren Träger enthält.

**12.** Zusammensetzung nach Anspruch 11, die zur parenteralen Verabreichung geeignet ist.

**Revendications**

**1.** Un acide désoxyribonucléique (ADN) bi-caténaire qui encode la protéine S humaine dans laquelle le bout codant est :

5'-R'-GCA AAT TCT

TTA CTT GAA GAA ACC AAA CAG GGT AAT CTT GAA AGA GAA TGC ATC GAA

GAA CTG TGC AAT AAA GAA GAA GCC AGG GAG GTC TTT GAA AAT GAC CCG

GAA ACG GAT TAT TTT TAT CCA AAA TAC TTA GTT TGT CTT CGC TCT TTT

CAA ACT GGG TTA TTC ACT GCT GCA CGT CAG TCA ACT AAT GCT TAT CCT

GAC CTA AGA AGC TGT GTC AAT GCC ATT CCA GAC CAG TGT AGT CCT CTG

CCA TGC AAT GAA GAT GGA TAT ATG AGC TGC AAA GAT GGA AAA GCT TCT

TTT ACT TGC ACT TGT AAA CCA GGT TGG CAA GGA GAA AAG TGT GAA TTT

GAC ATA AAT GAA TGC AAA GAT CCC TCA AAT ATA AAT GGA GGT TGC AGT

CAA ATT TGT GAT AAT ACA CCT GGA AGT TAC CAC TGT TCC TGT AAA AAT

GGT TTT GTT ATG CTT TCA AAT AAG AAA GAT TGT AAA GAT GTG GAT GAA

TGC TCT TTG AAG CCA AGC ATT TGT GGC ACA GCT GTG TGC AAG AAC ATC

CCA GGA GAT TTT GAA TGT GAA TGC CCC GAA GGC TAC AGA TAT AAT CTC

AAA TCA AAG TCT TGT GAA GAT ATA GAT GAA TGC TCT GAG AAC ATG TGT

GCT CAG CTT TGT GTC AAT TAC CCT GGA GGT TAC ACT TGC TAT TGT GAT

GGG AAG AAA GGA TTC AAA CTT GCC CAA GAT CAG AAG AGT TGT GAG GTT

GTT TCA GTG TGC CTT CCC TTG AAC CTT GAC ACA AAG TAT GAA TTA CTT

TAC TTG GCG GAG CAG TTT GCA GGG GTT GTT TTA TAT TTA AAA TTT CGT

TTG CCA GAA ATC AGC AGA TTT TCA GCA GAA TTT GAT TTC CGG ACA TAT

GAT TCA GAA GGC GTG ATA CTG TAC GCA GAA TCT ATC GAT CAC TCA GCG

TGG CTC CTG ATT GCA CTT CGT GGT GGA AAG ATT GAA GTT CAG CTT AAG

AAT GAA CAT ACA TCC AAA ATC ACA ACT GGA GGT GAT GTT ATT AAT AAT

GGT CTA TGG AAT ATG GTG TCT GTG GAA GAA TTA GAA CAT AGT ATT AGC

ATT AAA ATA GCT AAA GAA GCT GTG ATG GAT ATA AAT AAA CCT GGA CCC

CTT TTT AAG CCG GAA AAT GGA TTG CTG GAA ACC AAA GTA TAC TTT GCA

GGA TTC CCT CGG AAA GTG GAA AGT GAA CTC ATT AAA CCG ATT AAC CCT

CGT CTA GAT GGA TGT ATA CGA AGC TGG AAT TTG ATG AAG CAA GGA GCT

TCT GGA ATA AAG GAA ATT ATT CAA GAA AAA CAA AAT AAG CAT TGC CTG

GTT ACT GTG GAG AAG GGC TCC TAC TAT CCT GGT TCT GGA ATT GCT CAA

TTT CAC ATA GAT TAT AAT AAT GTA TCC AGT GCT GAG GGT TGG CAT GTA

```
AAT GTG ACC TTG AAT ATT CGT CCA TCC ACG GGC ACT GGT GTT ATG CTT

GCC TTG GTT TCT GGT AAC AAC ACA GTG CCC TTT GCT GTG TCC TTG GTG

GAC TCC ACC TCT GAA AAA TCA CAG GAT ATT CTG TTA TCT GTT GAA AAT

ACT GTA ATA TAT CGG ATA CAG GCC CTA AGT CTA TGT TCC GAT CAA CAA

TCT CAT CTG GAA TTT AGA GTC AAC AGA AAC AAT CTG GAG TTG TCG ACA

CCA CTT AAA ATA GAA ACC ATC TCC CAT GAA GAC CTT CAA AGA CAA CTT

GCC GTC TTG GAC AAA GCA ATG AAA GCA AAA GTG GCC ACA TAC CTG GGT

GGC CTT CCA GAT GTT CCA TTC AGT GCC ACA CCA GTG AAT GCC TTT TAT

AAT GGC TGC ATG GAA GTG AAT ATT AAT GGT GTA CAG TTG GAT CTG GAT

GAA GCC ATT TCT AAA CAT AAT GAT ATT AGA GCT CAC TCA TGT CCA TCA

GTT TGG AAA AAG ACA AAG AAT TCT-3'
```

dans laquelle R' est 5'

```
                        -ATG AGG GTC CTG GGT GGG CGC TGC GGG GCG CCG CTG

GCG TGT CTC CTC CTA GTG CTT CCC GTC TCA GAG GCA AAC CTT CTG TCA

AAG CAA CAG GCT TCA CAA GTC CTG GTT AGG AAG CGT CGT-3',
```

A est désoxyadényle,
G est désoxyguanyle,
C est désoxycytidyle, et
T est thymidyle.

**2.** Un acide désoxyribonucléique bi-caténaire qui encode un polypeptide qui a une activité de protéine S humaine dans laquelle le bout codant est :

```
                                                5'-GCA AAT TCT

TTA CTT GAA GAA ACC AAA CAG GGT AAT CTT GAA AGA GAA TGC ATC GAA

GAA CTG TGC AAT AAA GAA GAA GCC AGG GAG GTC TTT GAA AAT GAC CCG

GAA ACG GAT TAT TTT TAT CCA AAA TAC TTA GTT TGT CTT CGC TCT TTT

CAA ACT GGG TTA TTC ACT GCT GCA CGT CAG TCA ACT AAT GCT TAT CCT

GAC CTA AGA AGC TGT GTC AAT GCC ATT CCA GAC CAG TGT AGT CCT CTG

CCA TGC AAT GAA GAT GGA TAT ATG AGC TGC AAA GAT GGA AAA GCT TCT
```

42

```
TTT ACT TGC ACT TGT AAA CCA GGT TGG CAA GGA GAA AAG TGT GAA TTT
GAC ATA AAT GAA TGC AAA GAT CCC TCA AAT ATA AAT GGA GGT TGC AGT
CAA ATT TGT GAT AAT ACA CCT GGA AGT TAC CAC TGT TCC TGT AAA AAT
GGT TTT GTT ATG CTT TCA AAT AAG AAA GAT TGT AAA GAT GTG GAT GAA
TGC TCT TTG AAG CCA AGC ATT TGT GGC ACA GCT GTG TGC AAG AAC ATC
CCA GGA GAT TTT GAA TGT GAA TGC CCC GAA GGC TAC AGA TAT AAT CTC
AAA TCA AAG TCT TGT GAA GAT ATA GAT GAA TGC TCT GAG AAC ATG TGT
GCT CAG CTT TGT GTC AAT TAC CCT GGA GGT TAC ACT TGC TAT TGT GAT
GGG AAG AAA GGA TTC AAA CTT GCC CAA GAT CAG AAG AGT TGT GAG GTT
GTT TCA GTG TGC CTT CCC TTG AAC CTT GAC ACA AAG TAT GAA TTA CTT
TAC TTG GCG GAG CAG TTT GCA GGG GTT GTT TTA TAT TTA AAA TTT CGT
TTG CCA GAA ATC AGC AGA TTT TCA GCA GAA TTT GAT TTC CGG ACA TAT
GAT TCA GAA GGC GTG ATA CTG TAC GCA GAA TCT ATC GAT CAC TCA GCG
TGG CTC CTG ATT GCA CTT CGT GGT GGA AAG ATT GAA GTT CAG CTT AAG
AAT GAA CAT ACA TCC AAA ATC ACA ACT GGA GGT GAT GTT ATT AAT AAT
GGT CTA TGG AAT ATG GTG TCT GTG GAA GAA TTA GAA CAT AGT ATT AGC
ATT AAA ATA GCT AAA GAA GCT GTG ATG GAT ATA AAT AAA CCT GGA CCC
CTT TTT AAG CCG GAA AAT GGA TTG CTG GAA ACC AAA GTA TAC TTT GCA
GGA TTC CCT CGG AAA GTG GAA AGT GAA CTC ATT AAA CCG ATT AAC CCT
CGT CTA GAT GGA TGT ATA CGA AGC TGG AAT TTG ATG AAG CAA GGA GCT
TCT GGA ATA AAG GAA ATT ATT CAA GAA AAA CAA AAT AAG CAT TGC CTG
GTT ACT GTG GAG AAG GGC TCC TAC TAT CCT GGT TCT GGA ATT GCT CAA
TTT CAC ATA GAT TAT AAT AAT GTA TCC AGT GCT GAG GGT TGG CAT GTA
AAT GTG ACC TTG AAT ATT CGT CCA TCC ACG GGC ACT GGT GTT ATG CTT
GCC TTG GTT TCT GGT AAC AAC ACA GTG CCC TTT GCT GTG TCC TTG GTG
GAC TCC ACC TCT GAA AAA TCA CAG GAT ATT CTG TTA TCT GTT GAA AAT
ACT GTA ATA TAT CGG ATA CAG GCC CTA AGT CTA TGT TCC GAT CAA CAA
TCT CAT CTG GAA TTT AGA GTC AAC AGA AAC AAT CTG GAG TTG TCG ACA
CCA CTT AAA ATA GAA ACC ATC TCC CAT GAA GAC CTT CAA AGA CAA CTT
GCC GTC TTG GAC AAA GCA ATG AAA GCA AAA GTG GCC ACA TAC CTG GGT
GGC CTT CCA GAT GTT CCA TTC AGT GCC ACA CCA GTG AAT GCC TTT TAT
AAT GGC TGC ATG GAA GTG AAT ATT AAT GGT GTA CAG TTG GAT CTG GAT
GAA GCC ATT TCT AAA CAT AAT GAT ATT AGA GCT CAC TCA TGT CCA TCA
GTT TGG AAA AAG ACA AAG AAT TCT-3'
```

dans laquelle
A est désoxyadényle,
G est désoxyguanyle,
C est désoxycytidyle et
T est thymidyle.

3. Un vecteur d'ADN recombinant qui comprend l'acide désoxyribonucléique bi-caténaire de la revendication 1 ou 2.

4. Le vecteur de la revendication 3 qui est un plasmide pShd produit par ligation du fragment de restriction FnuDII-EcoRV contenant la protéine S d'environ 2,7 Kb du plasmide pHHS-IIa (NRRL B-18071) à un plasmide phd traité par Klenow et digéré par BclI (tel que montré à la figure 14).

5. Un vecteur de la revendication 3 qui est le plasmide pHHS-IIa (NRRL B-18071).

6. Des cellules Escherichia coli transformées avec le vecteur des revendications 3, 4 ou 5.

7. Une culture de cellules transformees avec le vecteur de la revendication 3 ou 4.

8. La culture de la revendication 7 dans laquelle les cellules sont des cellules de rein humain 293.

9. Un procédé pour produire la protéine S humaine qui comprend la mise en culture de la culture de la revendication 7 ou 8 dans des conditions permettant l'expression de la protéine S humaine et la récupération de la protéine exprimee.

10. Protéine S humaine produite par le procédé de la revendication 9.

11. Composition pharmaceutique comprenant la protéine S humaine produite par le procédé de la revendication 9 en mélange avec des porteurs pharmaceutiquement acceptables.

12. Composition de la revendication 11 qui est utilisable pour l'administration parentérale.

44

# FIG. I

Restriction Site and Function Map of
Plasmid pShd
(11.5 kb)

# FIG.2

Restriction Site and Function Map
of Plasmid pHHS-IIa
(7.7 Kb)

# FIG.3

Restriction Site and Function Map of
Plasmid pBKE1
(7.9 kb)

# FIG.4

Restriction Site and Function Map of
Plasmid pBKneo1
(11.5 kb)

# FIG.5

Restriction Site and Function Map of
Plasmid pSV2cat
(5.015 kb)

# FIG.6

**Restriction Site and Function Map of
Plasmid pLPcat
(4.83 kb)**

# FIG.7

Restriction Site and Function Map of
Plasmid pBLcat
(6.09 kb)

# FIG.8
## Construction of Plasmid pL133

# FIG.9

**Restriction Site and Function Map of Plasmid pLPC (6.5 kb)**

# FIG.IO

**Restriction Site and Function Map of
Plasmid pLPC4
(11.7 kb)**

# FIG.II

Restriction Site and Function Map of
Plasmid pSV2hyg
(5.24 kb)

# FIG.12

Restriction Site and Function Map of
Plasmid pLPChyg1
(8.3 kb)

# FIG.13

Restriction Site and Function Map of
Plasmid pLPChd1
(10.23 kb)

# FIG.14

Restriction Site and Function Map of
Plasmid phd
(8.55 kb)